(19) Europäisches Patentamt
European Patent Office
Office européen des brevets

(11) EP 3 487 835 B1

(12) FASCICULE DE BREVET EUROPEEN

(45) Date de publication et mention de la délivrance du brevet:
**08.04.2020 Bulletin 2020/15**

(21) Numéro de dépôt: **17754393.1**

(22) Date de dépôt: **20.07.2017**

(51) Int Cl.:
***C07C 227/34*** *(2006.01)* ***C07C 229/34*** *(2006.01)*

(86) Numéro de dépôt international:
**PCT/FR2017/051992**

(87) Numéro de publication internationale:
**WO 2018/015677 (25.01.2018 Gazette 2018/04)**

(54) **PROCÉDÉ DE DÉDOUBLEMENT DE SELS DE BACLOFÈNE**

VERFAHREN ZUR AUFLÖSUNG VON BACLOFEN-SALZEN

METHOD FOR THE RESOLUTION OF BACLOFEN SALTS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.07.2016 FR 1657054**

(43) Date de publication de la demande:
**29.05.2019 Bulletin 2019/22**

(73) Titulaire: **Université de Rouen**
**76130 Mont-Saint-Aignan (FR)**

(72) Inventeurs:
- **COQUEREL, Gérard**
  **76130 Mont Saint Aignan (FR)**
- **MAHIEUX, Julien**
  **1612 Ecoteaux (CH)**
- **GENDRON, François-Xavier**
  **76190 Autretot (FR)**

(74) Mandataire: **Plasseraud IP**
**66, rue de la Chaussée d'Antin**
**75440 Paris Cedex 09 (FR)**

(56) Documents cités:
**WO-A1-97/22579 WO-A1-2011/073300**
**US-A- 6 022 409**

- **MAZZENGA G C ET AL: "The transdermal delivery of zwitterionic drugs I: the solubility of zwitterion salts", JOURNAL OF CONTROLLED RELEASE, ELSEVIER, AMSTERDAM, NL, vol. 16, no. 1-2, 1 juin 1991 (1991-06-01) , pages 77-88, XP025567249, ISSN: 0168-3659, DOI: 10.1016/0168-3659(91)90032-9 [extrait le 1991-06-01]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

### DOMAINE TECHNIQUE

La présente invention concerne le domaine du dédoublement de composés chiraux existant sous la forme de deux antipodes optiques (énantiomères), comme le Baclofène. The transdermal delivery of zwitterionic drugs I: the solubility of zwitterion salts",JOURNAL OF CONTROLLED RELEASE, ELSEVIER, AMSTERDAM, NL, vol. 16, no. 1-2, (1991-06-01), pages 77-88, divulgue le sel racémique de maléate de Baclofène

Plus particulièrement, l'invention concerne la préparation des énantiomères purs (R)(-) Baclofène et S(+)Baclofène, de nomenclature chimique acide (R)-4-amino-3-(4-chlorophényl)-butanoïque et acide (S)-4-amino-3-(4-chlorophényl)-butanoïque et de leur sel d'hydrogénomaléate.

Tout spécialement, la présente invention concerne le dédoublement des sels d'hydrogénomaléate du Baclofène racémique par cristallisation préférentielle et notamment par le procédé AS3PC (cristallisation préférentielle polythermique programmée et auto-ensemencée) ou le procédé ASPreCISE (cristallisation préférentielle auto-ensemencée induite par évaporation de solvant).

### ARRIÈRE-PLAN DE L'INVENTION

Le Baclofène racémique est représenté par la formule générale (I) ci-dessous :

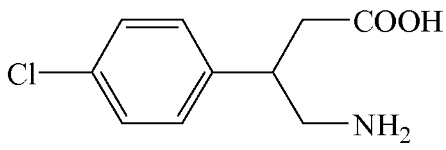
(I).

L'énantiomère pur (R)(-)Baclofène est représenté par la formule générale (II) ci-dessous :

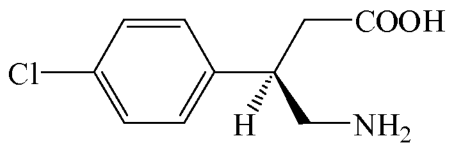
(II).

L'énantiomère pur (S)(-)Baclofène est représenté par la formule générale (III) ci-dessous :

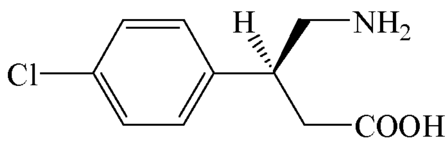
(III).

Le Baclofène, également connu sous le nom de Liorésal, est un médicament utilisé comme myorelaxant pour traiter des contractures douloureuses qui accompagnent la sclérose en plaques et certaines paralysies.

En France, l'Agence Nationale de Sécurité du Médicament et des Produits de Santé (ANSM) a récemment délivré une recommandation temporaire d'utilisation du Baclofène pour le traitement de la dépendance alcoolique.

Dans son utilisation thérapeutique actuelle, cette molécule est administrée sous forme d'un mélange racémique. L'énantiomère R(-) étant trois fois plus actif que l'énantiomère S(+), il apparait intéressant, surtout pour de longs traitements, de ne prescrire que la seule configuration absolue R(-) la plus active. De la sorte, il y aura moins de sous-produits dans l'organisme et le dosage pourra être réduit tout en préservant le bénéfice de l'activité.

Pour produire la forme R(-), les méthodes décrites dans la littérature mettent en œuvre soit une synthèse asymétrique à partir d'un mélange racémique ou d'un composé prochiral avec des catalyseurs, soit une synthèse énantiosélective à partir d'un réactif chiral.

Par exemple, il est possible d'utiliser des catalyseurs enzymatique, comme des bactéries de type *Rhodococcus sp.,* tel que décrit dans M.X. Wang, S.M. Zhao, Tetrahedron Lett. 2002, 43, 6617-6620, pour accéder au R(-)Baclofène selon le schéma suivant :

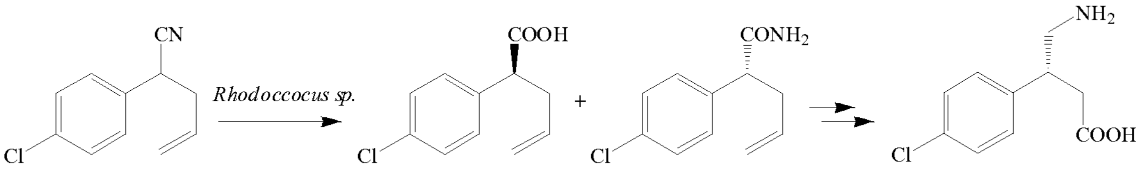


racémique acide (R) amide (S) R(-)Baclofène.

On connait également, dans Canadian Journal of Chemistry, 1994, 72(11), 2312-2317, une voie de synthèse asymétrique du R(-)Baclofène mettant en œuvre une désymétrisation d'un glutarate prochiral par la chymotrypsine selon le schéma suivant :

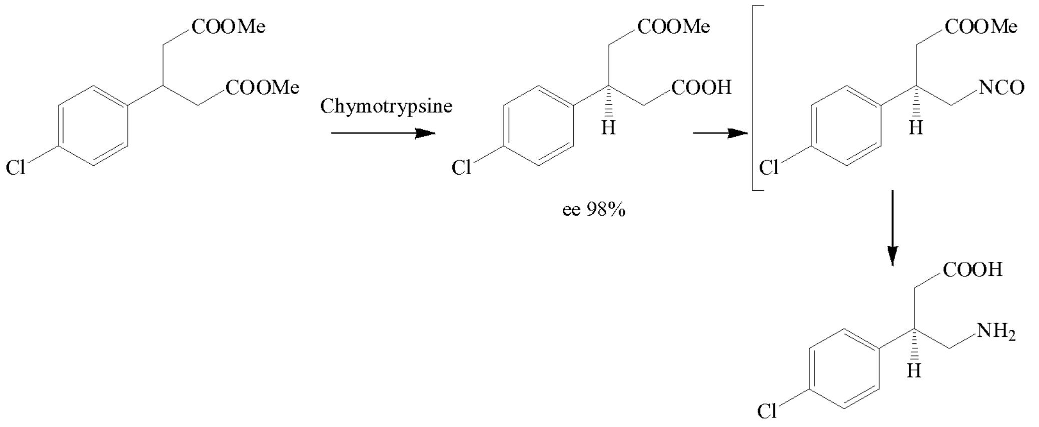


R(-)Baclofène.

Enfin, la demande de brevet WO 94/02443 décrit une synthèse énantiosélective du R(-)Baclofène à partir d'un dérivé de l'acide S-pyroglutamique selon le schéma suivant :

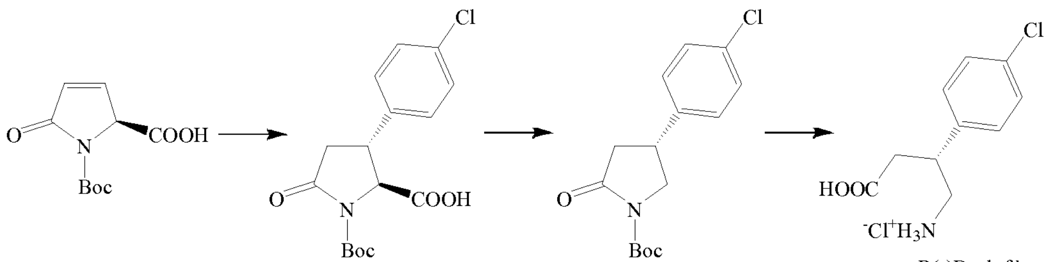

R(-)Baclofène.

Toutefois, ces méthodes mettent en œuvre des réactifs couteux et difficilement industrialisables. De plus, le rendement final en R(-)Baclofène est assez faible. En outre, du fait du nombre d'étapes de synthèse, le produit final est contaminé par des impuretés qui doivent être éliminées par des étapes de purification afin d'obtenir un produit suffisamment pur pour être administré comme médicament.

Dans ce contexte, les inventeurs ont mis au point un procédé permettant de séparer les énantiomères du Baclofène en partant d'un mélange racémique. Ce procédé est avantageusement industrialisable et ne requiert pas l'utilisation de dérivés chiraux. De plus, les étapes du procédé de la présente invention sont faciles à mettre en œuvre et il n'y a aucune perte de matière première grâce aux recyclages successifs.

Ce but est atteint grâce à l'application du procédé de cristallisation préférentielle au Baclofène racémique sous forme de sel. Ainsi, l'invention se rapporte tout particulièrement à l'application à un sel racémique d'hydrogénomaléate de Baclofène, du dédoublement par cristallisation préférentielle en chacun de ses énantiomères, permettant d'obtenir l'eutomère R(-)Baclofène dans une forme énantiomériquement et chimiquement pure.

Le sel racémique d'hydrogénomaléate de Baclofène peut être résolu par n'importe quel type de cristallisation préférentielle, notamment les plus avantageuses, c'est à dire les procédés auto-ensemencés.

En particulier, le procédé de cristallisation préférentielle AS3PC a fait l'objet d'un développement tout à fait original excluant l'utilisation contraignante de germes de cristallisation (i.e. sans ensemencement). Ce procédé est décrit

par exemple dans les brevets et demandes de brevet suivants: FR 2 710 337, WO 95/08522, EP 0 720 595 et US 6,022,409 et dans «G. Coquerel, Preferential Crystallization in Topic in Current Chemistry, Novel Optical Resolution Technologies, Springer, Berlin, Heidelberg, Eds. K. Sakai, N. Hirayama and R. Tamura », 2007, 269, 1-51. Ce procédé est désigné « AS3PC », pour "Auto-Seeded Programmed Polythermic Preferential Crystallization" .

Un autre procédé de cristallisation préférentielle autoensemencé est décrit dans la demande de brevet WO 2011/07330. Ce procédé est désigné par l'acronyme: "ASPreCISE" pour "Auto-Seeded PREferential Crystallization Induced by Solvent Evaporation".

Les procédés de cristallisation préférentielle reposent sur la cristallisation stéréosélective alternée des deux énantiomères (R) et (S), d'une même espèce chimique racémique cristallisant sous forme de conglomérat, dans un médium qui peut être un solvant ou un mélange de solvants ou un ensemble de constituants incluant le ou les solvants, et ce pour une gamme donnée de température $\Delta T$. Dans cette gamme de température, ce mélange racémique, en équilibre thermodynamique avec sa solution saturée, est constitué de deux types de cristaux ne contenant chacun que des molécules de même configuration absolue.

La connaissance de ces équilibres hétérogènes énantiomère (R) - énantiomère (S) - solvant fournit des données mises à profit pour réaliser un dédoublement efficace par cristallisation préférentielle.

Les études menées par la Demanderesse montrent que le Baclofène racémique ne cristallise pas sous forme d'un conglomérat. Cela signifie que l'on ne peut pas appliquer le procédé de cristallisation préférentielle AS3PC ou ASPreCISE ou tout autre procédé de cristallisation préférentielle.

Dans l'optique de la mise en place d'un tel procédé, une recherche d'un sel de Baclofène permettant la discrimination chirale a été effectuée par addition d'un co-formeur, tel qu'un acide, une base ou un métal alcalin. La liste complète des différents co-formeurs testés est présentée dans le Tableau 1 ci-dessous :

Tableau 1

| Co-formeurs : acides, bases ou métaux alcalins (les composés en **gras** sont ceux pour lesquels une nouvelle phase est observée, la nouvelle phase étant un co-cristal comprenant le Baclofène et le co-formeur) | | |
|---|---|---|
| acide 4-nitrobenzoïque | acide cholique | acide adipique |
| acide 3,4-dichlorobenzoÏque | acide 3-bromo-4-nitrobenzoïque | acide 4-hydroxybenzènesulfonique |
| acide 4-fluoro-3-nitrobenzoïque | acide 3-fluoro-4-nitrobenzoïque | acide dichloroacétique |
| acide 2-chloro-4-nitrobenzoïque | acide 2,5-dinitrobenzoïque | acide tétrafluoroborique |
| acide 2,4-dihydroxybenzoïque | acide 3-méthoxy-4-nitrobenzoïque | acide trifluorométhanesulfonique |
| acide 4-hydroxybenzoïque | acide 2-chloro-3,5-dinitrobenzoïque | acide acétique |
| acide 4-chloro-3,5-dinitrobenzoïque | acide 2-méthyl-3,5-dinitrobenzoïque | acide 2,4-diaminobenzènesulfonique |
| acide 3,5-dinitro-4-toluique | acide 3,5-dinitrosalicylique | **acide méthanesulfonique** |
| acide 4-méthyl-3-nitrobenzoïque | acide 3-bromo-5-nitrobenzoïque | acide 4-nitrobenzènesulfonique |
| acide 3-nitro-5-(trifluorométhyl) benzoïque | acide salicylique | **acide trichloroacétique** |
| acide 3,4-dinitrobenzoïque | acide hydrocinnamique | **acide benzènesulfonique** |
| acide citraconique | acide 5-chloro-2-nitrobenzoïque | acide 3,5-diamino-2,4,6-triméthylbenzènesulfonique |
| acide 3,5-dinitrobenzoïque | acide 2,5-dichlorobenzoïque | **acide 1,2-phénylènediacétique** |
| acide *o*-toluique | acide fumarique | acide 2,5-diaminobenzènesulfonique |
| acide 3-nitrobenzoïque | acide 2-phénylbutyrique | acide bromoacétique |
| acide 4-chloro-3-nitrobenzoïque | acide 2-tétrahydrofolique | acide éthanesulfonique |
| acide 3-méthyl-4-nitrobenzoïque | acide *trans*-3,4-diméthoxycinnamique | acide méthanesulfonique |
| **acide oxalique** | acide 3-phénylbutyrique | **acide méthoxyacétique** |

(suite)

| Co-formeurs : acides, bases ou métaux alcalins (les composés en **gras** sont ceux pour lesquels une nouvelle phase est observée, la nouvelle phase étant un co-cristal comprenant le Baclofène et le co-formeur) | | |
|---|---|---|
| acide 4-nitrobenzoïque | acide cholique | acide adipique |
| acide méthylsulfamique | **acide 4-chlorobenzène-sulfonique** | hexaméthylènetétramine |
| acide stéarique | acide butyl-éthyl-hydroxy-propionique | diphénylamine |
| acide undécanedioïque | acide *trans*-cinnamique | tétrahydrofurfurylamine |
| acide *cis,cis*-muconique | acide glutarique | tert-butylamine |
| acide 2,4-diaminobenzenesulfonique | acide isophtalique | benzylamine |
| **acide glycolique** | acide itaconique | n-butylamine |
| acide pimélique | acide malonique | éthylènediamine |
| acide tétradécanedioïque | acide n-butyrique | N,N'-dibenzyléthylènediamine |
| acide mucique | acide *p*-tolyacétique | éthanolamine |
| acide subérique | acide propionique | ammoniaque |
| acide sébacique | **acide 1H-benzimidazol-2-sulfonique** | triéthanolamine |
| acide dodécanadioïque | acide 1-naphtalène-sulfonique | hydroxyde de potassium |
| acide urique | **acide 3-pyridinesulfonique** | hydroxyde de calcium |
| acide succinique | acide chloroacétique | hydroxyde de magnésium |
| acide borique | **acide 1-hydroxy-2-naphtoïque** | hydroxyde d'aluminium |
| acide *p*-toluènesulfonique monohydraté | acide 1-propanesulfonique | hydroxyde de strontium |
| acide citrique | acide iodoacétique | hydroxyde de lithium monohydraté |
| acide nitrique | **acide chlorhydrique** | hydroxyde de sodium |
| acide sulfurique | acide bromique | formiate de rubidium hydraté |
| acide phosphorique | acide iodhydrique | acide trifluoroacétique |
| acide sulfamique | acide perchlorique | acide pyrophosphorique |

Par contre, de manière tout à fait inattendue, la Demanderesse a trouvé, que le Baclofène formait avec l'acide maléique un sel qui cristallise sans formation de solvate dans la plupart des solvants usuels et sans formation d'un eutectique en milieu fondu. Ce dérivé salin présente en outre l'avantage d'être un sel pharmaceutiquement acceptable et peu onéreux. De façon surprenante, ce sel présente une discrimination chirale quasi-totale à température ambiante mais aucune discrimination chirale à haute température. Cette propriété a été utilisée pour dédoubler le mélange racémique avec un rendement optimal par application répétée de la cristallisation préférentielle. De plus, le procédé de dédoublement peut être mis en œuvre dans l'eau en utilisant le dédoublement auto-ensemencé AS3PC pour une plus grande facilité d'exploitation à l'échelle industrielle

**EXPOSÉ DE L'INVENTION**

La présente invention a pour objet un sel racémique d'hydrogénomaléate de Baclofène (Bahma) présentant un point de fusion/décomposition de 164±1°C.

Un autre objet de l'invention est l'utilisation du sel racémique d'hydrogénomaléate de Baclofène pour dédoubler les énantiomères (S) et (R) du Baclofène.

La présente invention a également pour objet un procédé de dédoublement des énantiomères (S) et (R) du Baclofène, dans lequel le Baclofène racémique est transformé en sel racémique d'hydrogénomaléate de Baclofène en

présence d'acide maléique et dans lequel ledit sel est dédoublé par cristallisation préférentielle pour séparer les deux énantiomères (S) et (R).

La présente invention a également pour objet un procédé de purification énantiomérique de sels d'hydrogénomaléate de Baclofène comprenant la recristallisation de sels d'hydrogénomaléate de Baclofène dans un solvant.

## DESCRIPTION DETAILLÉE

Le sel racémique d'hydrogénomaléate de Baclofène objet de la présente invention présente un point de fusion/décomposition de 164±1°C. Le point de fusion/décomposition est mesuré par calorimétrie différentielle à balayage (DSC) selon la méthode décrite ci-après. Le terme « Bahma » utilisé dans la présente demande désigne « hydrogénomaléate de Baclofène ».

Le point de fusion/décomposition correspond au point de fusion du sel Bahma qui est suivi ou s'accompagne d'une décomposition du sel Bahma. En effet, le sel Bahma peut subir une ou plusieurs réactions de décomposition, par exemple la formation de l'anhydride maléique ou l'estérification du baclofène ou une autre réaction de décomposition.

Ledit sel répond à la formule $[C_{10}H_{13}ClNO_2]^+,[C_4H_3O_4]^-$. Ainsi, la fonction amine du Baclofène est protonée et il y a une seule molécule de Baclofène et une seule molécule d'hydrogénomaléate dans l'unité asymétrique. La masse molaire du sel est 329.73 $g.mol^{-1}$.

Ledit sel peut notamment être obtenu en dissolvant un mélange racémique de Baclofène et d'acide maléique dans des proportions stœchiométriques dans un solvant ou mélange de solvants.

La réaction de salification peut notamment être mise en œuvre dans un solvant choisi parmi l'acétone, l'eau, le méthanol, l'azéotrope eau/n-propanol et leurs mélanges.

Afin d'assurer une bonne cristallisation, il est avantageux de dissoudre le Baclofène et l'acide maléique dans un volume de solvant le plus faible possible. De plus, pour atteindre une solution homogène, le mélange peut être chauffé.

Après dissolution des solides, on laisse la solution revenir à température ambiante et les cristaux se forment par évaporation du solvant en quelques jours.

Les monocristaux obtenus présentent un faciès caractéristique avec des zones de réflexion totale, le long de l'axe de plus grand développement du cristal, tel que présenté à la Figure 1.

Les sels (R) et (S) d'hydrogénomaléate de Baclofène de la présente invention présentent une solution solide totale à haute température, notamment à une température supérieure à 150°C. En effet, le diagramme de phase des deux énantiomères de la Figure 2 montre qu'il existe un domaine monophasé unique, c'est-à-dire une solution solide totale, au-delà de 150°C. En dessous de cette température, qui correspond exactement au point critique maximum de la démixtion à l'état solide du mélange racémique, il y une discrimination chirale à l'état solide qui s'amplifie à mesure que la température est abaissée.

De façon inattendue, la Figure 2 montre également qu'il existe une discrimination chirale quasi-totale à une température inférieure ou égale à 70°C. Ainsi, en dessous de 70°C, le domaine monophasé est très faible en composition, c'est-à-dire moins de <1% de l'autre énantiomère, de chaque côté du diagramme de phase binaire. Cette large lacune de démixtion offre une discrimination chirale à l'état solide très importante qui peut être mise à profit pour mettre en œuvre un dédoublement par cristallisation préférentielle ou une purification énantiomérique préparative i.e. sans perte d'excès énantiomérique.

Ce comportement était d'autant moins anticipé que le Baclofène et l'acide maléique offrent de multiples possibilités de liaisons hydrogènes dirigées peu propices à la formation de solution solide. Ce cas très rare de solution solide totale avec démixtion à l'état solide se différencie des cas plus classiques de conglomérats avec solution solide partielle dont le diagramme de phase est représenté à la Figure 3.

Il est à noter que de nombreux sels de Baclofène autres que Bahma ont été étudiés (cf. Tableau 1 ci-dessus). Toutefois, leurs diagrammes de phase binaires, semblables à celui représenté à la Figure 4, ne présentent pas de solution solide totale ni de discrimination chirale ce qui ne permet pas d'envisager un dédoublement par cristallisation préférentielle.

Ainsi, de par son comportement spécifique, l'hydrogénomaléate de Baclofène est, a priori, tout à fait approprié à une utilisation pour dédoubler les énantiomères (S) et (R) du Baclofène.

L'invention a également pour objet un procédé de dédoublement des énantiomères (S) et (R) du Baclofène, dans lequel on transforme le Baclofène racémique en sel racémique d'hydrogénomaléate de Baclofène en présence d'acide maléique. Le sel racémique de Bahma obtenu est ensuite dédoublé par cristallisation préférentielle pour séparer les deux énantiomères (S) et (R).

Le dédoublement du sel racémique de Bahma peut notamment être réalisé par cristallisation préférentielle autoensemencée (AS3PC ou ASPreCISE) ou par cristallisation préférentielle ensemencée, de préférence par cristallisation préférentielle autoensemencée.

Selon un mode de réalisation particulier du procédé de la présente invention, la cristallisation préférentielle est mise en œuvre avec un solvant choisi parmi un solvant alcoolique, une solution aqueuse, une solution aqueuse acide

et les mélanges de ceux-ci.

Des exemples de solvants alcooliques pouvant être utilisés sont le méthanol, l'éthanol, le n-propanol et leurs mélanges, en particulier le n-propanol.

Selon un mode de réalisation particulier, le solvant est un mélange azéotropique de n-propanol et d'eau.

Selon un mode de réalisation préféré, la cristallisation préférentielle est mise en œuvre avec une solution aqueuse acide, l'acide étant choisi parmi l'acide chlorhydrique, l'acide acétique, l'acide nitrique, de préférence une solution aqueuse d'acide chlorhydrique, plus préférentiellement une solution aqueuse d'acide chlorhydrique à 2 mol/L.

En effet, la solubilité de Bahma dans une solution aqueuse acidifiée est supérieure à celle de Bahma dans de l'eau ou dans un mélange azéotropique de n-propanol et d'eau. Cette meilleure solubilité permet d'augmenter la productivité de la cristallisation préférentielle.

Selon un mode de réalisation particulier, la cristallisation préférentielle est autoensemencée et comprend les étapes suivantes :

a) on prépare un volume V d'une solution saturée de sel racémique de Bahma dans un solvant à une température $T_L$ ;
b) on ajoute au moins 5% en poids du premier énantiomère Bahma à récupérer par rapport au poids du sel racémique de Bahma ;
c) on chauffe le mélange à une température $T_B = T_L + \Delta T$;
d) on applique une loi de programmation de refroidissement au mélange de $T_B$ à $T_F$, $T_F$ étant inférieur à $T_B$, telle que le mélange garde une faible sursaturation qui privilégie la croissance du premier énantiomère Bahma présent sous forme de cristaux, tout en interdisant la nucléation spontanée du second énantiomère Bahma dissous dans la solution ;
e) on récolte les cristaux du premier énantiomère Bahma à la température $T_F$ ;
f) on ajoute au mélange sensiblement la même masse de sel racémique de Bahma que la masse de la récolte réalisée à l'étape précédente, on complète avec du solvant pour atteindre le volume V et on porte le nouvel ensemble à la température $T_B$ ;
g) on maintient la température $T_B$ pendant un temps t afin de permettre au système de revenir à l'équilibre thermodynamique ;
h) on applique la même loi de programmation de refroidissement qu'à l'étape (d) au mélange préparé à l'étape (g) contenant le second énantiomère Bahma, de sorte que le mélange garde une faible sursaturation pendant la cristallisation afin de privilégier la croissance du second énantiomère Bahma présent sous forme de cristaux tout en interdisant la nucléation spontanée du premier énantiomère Bahma présent dans la solution ;
j) on récolte les cristaux du second énantiomère Bahma à la température $T_F$ ;
j) on ajoute au mélange sensiblement la même masse de sel racémique de Bahma que la masse de la récolte réalisée à l'étape précédente, on complète avec du solvant pour atteindre le volume V et on porte le nouvel ensemble à la température $T_B$ ;
k) on maintient la température $T_B$ pendant un temps t afin de permettre au système de revenir à l'équilibre thermodynamique ;
l) on répète les étapes (d) à (k) pour obtenir successivement l'un puis l'autre des deux énantiomères.

Dans le procédé ci-dessus, le solvant est tel que décrit précédemment, notamment un mélange azéotropique de n-propanol et d'eau ou une solution aqueuse acide. Le volume V de solvant utilisé pour obtenir une solution saturée est déterminé en fonction de la quantité de sel racémique de Bahma à dédoubler et de la solubilité du sel racémique de Bahma dans le solvant choisi.

À l'étape (b) du procédé ci-dessus, la quantité du premier énantiomère Bahma ajoutée est d'au moins 5% en poids par rapport au poids du sel racémique de Bahma dissous dans le solvant, en particulier de 5 à 15% en poids, plus particulièrement de 5 à 10% en poids.

À l'étape (c) du procédé ci-dessus, la température $T_L$ correspond à la température de dissolution du mélange racémique seul dans le solvant selon l'étape (a). Selon un mode de réalisation particulier, la température $T_L$ va de 30 à 70°C, de préférence $T_L$ va de 40 à 60°C, plus préférentiellement $T_L$ est 50°C.

À l'étape (c) du procédé ci-dessus la température $T_B$ correspond à une température légèrement supérieure à la température de dissolution du mélange racémique $T_L$. Ainsi, la température $T_B = T_L + \Delta T$ où $\Delta T$ va de 1°C à 10°C, en particulier de 2°C à 7°C, plus particulièrement de 3°C à 5°C.

Avantageusement, dans les étapes (d) et (h) du procédé ci-dessus, on adapte pendant toute la durée de la croissance cristalline une vitesse d'agitation légèrement croissante en fonction du temps de façon à ce que celle-ci soit suffisamment lente pour favoriser une croissance du premier ou du second énantiomère Bahma, en évitant de générer une nucléation non maîtrisée et l'attrition de cristaux.

Dans le procédé ci-dessus, la température $T_F$ dépend de la quantité de sel racémique de Bahma que l'on souhaite dédoubler. Selon un mode de réalisation particulier, la température $T_F$ va de 20 à 40°C, de préférence $T_F$ va

de 25 à 35°C, plus préférentiellement $T_F$ est 30°C.

Dans le procédé ci-dessus, le temps t dépend de la quantité de sel racémique de Bahma que l'on souhaite dédoubler. Selon un mode de réalisation particulier, le temps t est supérieur à 20 min, de préférence de 25 min à 45 min, plus préférentiellement t est 30 min.

Aux étapes (e) et (i) du procédé ci-dessus, les cristaux du premier énantiomère Bahma et les cristaux du second énantiomère Bahma sont récoltés par filtration ou centrifugation.

Les cristaux du premier énantiomère Bahma et les cristaux du second énantiomère Bahma obtenus avec le procédé objet de la présente invention peuvent notamment présenter un excès énantiomérique supérieur à 80%. Lesdits cristaux peuvent être recristallisés pour obtenir un excès énantiomériques proche de 100%, notamment selon le procédé de purification énantiomérique selon l'invention décrit ci-après. Un solvant approprié pour la recristallisation est un solvant choisi parmi l'acétone, l'eau, le méthanol, l'azéotrope eau/n-propanol et leurs mélanges.

Selon un deuxième mode de réalisation, la cristallisation préférentielle est ensemencée et comprend les étapes suivantes :

a) on prépare une première solution homogène composée du sel racémique de Bahma, d'un excès du premier énantiomère de Bahma à récupérer et d'un solvant, dont le point figuratif I, défini par les variables concentration et température $T_I$ ($T_I > T_{HOMO}$), se situe dans le domaine monophasé composé de la solution sous-saturée ;
b) on applique une loi de programmation de refroidissement au mélange monophasé ;
c) lorsque le mélange atteint une température inférieure à la température $T_{HOMO}$, on ensemence la solution avec des germes énantiomériquement purs du premier énantiomère Bahma à récupérer ;
d) on adapte pendant toute la durée de la croissance cristalline une vitesse d'agitation légèrement croissante en fonction du temps de façon à ce que celle-ci soit suffisamment lente pour favoriser une croissance du premier énantiomère Bahma ;
e) on récolte les cristaux du premier énantiomère Bahma ;
f) on ajoute au mélange la même masse de sel racémique de Bahma que la masse de la récolte réalisée à l'étape précédente, et on porte le nouvel ensemble à la température $T_I$ ($T_I > T_{HOMO}$), le point I' se situant dans le domaine monophasé ;
g) on applique la même loi de programmation de refroidissement qu'à l'étape (b) au mélange monophasé préparé à l'étape (f) contenant le second énantiomère, de sorte que le mélange garde une faible sursaturation pendant la cristallisation afin de privilégier la croissance du second énantiomère Bahma lors de l'ensemencement;
h) lorsque le mélange atteint une température inférieure à la température $T_{HOMO}$, on ensemence la solution avec des germes énantiomériquement purs du second énantiomère Bahma ;
i) on adapte pendant toute la durée de la croissance cristalline de l'étape précédente, une vitesse d'agitation légèrement croissante en fonction du temps de façon à ce que celle-ci soit, suffisamment lente pour favoriser la croissance de ce second énantiomère Bahma ;
j) on récolte les cristaux du second énantiomère Bahma ;
k) on ajoute au mélange la même masse de sel racémique de Bahma que la masse de la récolte réalisée à l'étape précédente, pour obtenir une solution dont la composition est identique à celle de la solution initiale ;
l) on répète les étapes (b) à (k) pour obtenir successivement l'un puis l'autre des deux énantiomères.

La présente invention a également pour objet un procédé de purification énantiomérique de sels de Bahma comprenant la recristallisation de sels de Bahma dans un solvant. Le solvant peut notamment être choisi parmi acétone, eau, méthanol, azéotrope eau/n-propanol et leurs mélanges.

Le procédé de purification énantiomérique de la présente invention peut notamment être mis en œuvre à la suite du procédé de dédoublement des énantiomères du Baclofène selon la présente invention décrit ci-dessus.

Le procédé de purification énantiomérique de la présente invention repose sur l'exploitation du diagramme de phase ternaire comprenant le domaine des solutions solides et les solubilités du système {Solvant - Enantiomère (R) de Bahma - Enantiomère (S) de Bahma}, représenté à la Figure 7.

Cette figure représente une coupe isotherme et isobare du diagramme de phase ternaire entre les sels des 2 énantiomères de Bahma et un solvant, la température choisie permettant la forte discrimination chirale entre les 2 énantiomères. En partant d'un mélange de sels d'énantiomères (R) et (S) de Bahma de composition I connue, qui peut notamment être un mélange d'énantiomères (R) et (S) obtenu lors du procédé de cristallisation préférentielle selon la présente invention, et en ajoutant du solvant, on traverse différents domaines constitués de phases ayant chacune une composition et une nature différente :

**I** $\rightarrow$ **$H_0$** : domaine triphasé constitué des 2 énantiomères de Bahma sous forme solide (ssR et ssS) et de la solution racémique saturée (s.s.r.) ;

**$H_0$** $\rightarrow$ **G** : domaine biphasé constitué d'un sel enrichi en l'énantiomère (R) de Bahma (ssR) sous forme solide et de

sa solution saturée (sol.sat.), la proportion en cristaux d'énantiomère (R) de Bahma diminuant tout au long du segment [$H_0$G], le point $H_0$ étant le point le plus riche en cristaux d'énantiomère (R) de Bahma ;
**G** → **F** : domaine monophasé constitué d'une solution sous-saturée (U.S.S).

Ainsi, en connaissant précisément la composition initiale du mélange **I** et sa masse, les domaines de solutions solides et la solubilité du mélange racémique, on peut déterminer avec précision la composition du point $\mathbf{H_0}$, qui permet, par filtration, de séparer le sel enrichi en l'énantiomère (R) de Bahma sous forme solide, représenté par le point $\mathbf{P_0}$, de la solution racémique $\mathbf{L_0}$ saturée. Ce point $\mathbf{H_0}$ étant atteint par ajout d'un volume $\mathbf{V_{H0}}$ de solvant ou une masse $\mathbf{m_{H0}}$ de solvant au mélange **I.**

On peut également ajouter un volume de solvant $\mathbf{V_{H1}}$ légèrement supérieur à $\mathbf{V_{H0}}$ ($V_{H1} = V_{H0} + \Delta V$) ou une masse de solvant $\mathbf{m_{H1}}$ légèrement supérieure à $\mathbf{m_{H0}}$ ($m_{H1} = m_{H0} + \Delta m$) afin d'atteindre le point de composition $\mathbf{H_1}$**.** Après filtration on sépare le sel enrichi en l'énantiomère (R) de Bahma sous forme solide, représenté par le point $\mathbf{P_1}$**,** de la solution racémique $\mathbf{L_1}$ saturée. L'ajout de cette quantité de solvant $\mathbf{V_{H1}}$ ou $\mathbf{m_{H1}}$ permet l'obtention d'un sel comprenant une plus forte proportion d'énantiomère (R) de Bahma que celui obtenu en ajoutant une quantité de solvant $\mathbf{V_{H0}}$ ou $\mathbf{m_{H0}}$**.** En revanche, le rendement en sel enrichi en ajoutant une quantité de solvant $\mathbf{V_{H1}}$ ou $\mathbf{m_{H1}}$ est plus faible que celui obtenu en ajoutant une quantité de solvant $V_{\mathbf{H0}}$ ou $\mathbf{m_{H0}}$ puisqu'une quantité plus importante d'énantiomère (R) de Bahma reste dissous dans la solution saturée. Plus la quantité de solvant $\Delta\mathbf{V}$ ou $\Delta\mathbf{m}$ est faible et plus la quantité d'énantiomère (R) de Bahma qui reste dissous dans la solution saturée est limitée.

La Figure 7 illustre le procédé de purification énantiomérique pour un mélange initialement enrichi en énantiomère (R) de Bahma, mais le procédé peut être appliqué symétriquement à un mélange enrichi en énantiomère (S) de Bahma.

Ainsi, selon un mode de réalisation préféré, le procédé de purification énantiomérique de sel de Bahma selon la présente invention comprend les étapes suivantes :

a) fournir un mélange solide d'énantiomères (R) et (S) de Bahma de composition connue représenté par le point **I** sur la coupe isotherme et isobare du diagramme de phase ternaire entre les 2 énantiomères de Bahma et un solvant ;
b) ajouter une quantité de solvant de façon à passer dans le domaine biphasé constitué d'un sel enrichi en un énantiomère de Bahma sous forme solide et de sa solution saturée dudit diagramme de phase ternaire ;
c) filtrer le mélange obtenu à l'étape b) pour obtenir le sel enrichi en un énantiomère de Bahma.

De préférence, la quantité de solvant ajoutée dans l'étape b) est le volume $\mathbf{V_{H0}}$ ou la masse $\mathbf{m_{H0}}$ de solvant qui permet d'atteindre le point $\mathbf{H_0}$ sur ledit diagramme de phase, ledit point $\mathbf{H_0}$ correspondant à l'intersection de la droite passant entre le point **I** et le point **F,** le point **F** étant le sommet du diagramme de phase correspondant au solvant pur, et de la droite $\mathbf{P_0}$-$\mathbf{L_0}$ (i.e. la conode) délimitant le domaine triphasé du domaine biphasé du sel enrichi en énantiomère de Bahma que l'on souhaite obtenir.

De préférence, la quantité de solvant ajoutée dans l'étape b) est le volume $\mathbf{V_{H1}} = \mathbf{V_{H0}} + \Delta\mathbf{V}$ ou la masse $\mathbf{m_{H1}} = \mathbf{m_{H0}} + \Delta\mathbf{m}$ de solvant qui permet d'atteindre le point $\mathbf{H_1}$ sur ledit diagramme de phase, ledit point $\mathbf{H_1}$ correspondant à l'intersection de la droite passant entre le point **I** et le point **F,** le point F étant le sommet du diagramme de phase correspondant au solvant pur, et de la droite $\mathbf{P_1}$-$\mathbf{L_1}$ (i.e. la conode).

Les sels de Bahma obtenus avec le procédé de dédoublement de la présente invention et/ou le procédé de purification énantiomérique de la présente invention peuvent être transformés en Baclofène ou en un sel de Baclofène autre que le sel de Bahma sans racémisation, c'est-à-dire sans perte d'excès énantiomérique. La transformation de sels de Bahma en Baclofène peut notamment être réalisée par ajout d'une base.

L'invention sera maintenant illustrée par les exemples non limitatifs suivants.

## FIGURES

**La Figure 1** est un cliché de microscopie optique d'un monocristal ayant un excès énantiomérique de 98.6% issu d'un sel racémique de Bahma préparé à l'exemple 1.
**La Figure 2** est le diagramme de phase binaire de Bahma obtenu par calorimétrie différentielle à balayage (DSC) (la courbe liquidus n'est pas présente étant donné que le sel Bahma se décompose pendant ou après sa fusion).
**La Figure 3** est un diagramme de phase binaire théorique d'un conglomérat présentant une solution solide partielle.
**La Figure 4** est un diagramme de phase binaire théorique d'un sel de Baclofène autre que le sel de Bahma avec la présence usuelle d'un composé racémique stœchiométrique.
**La Figure 5** représente la droite d'étalonnage tracée en faisant varier la concentration (C) d'un énantiomère pur de Bahma et en mesurant le pouvoir rotatoire spécifique (a) à une longueur d'onde de 365 nm dans l'azéotrope eau/n-propanol à 25°C.

**La Figure 6** représente le diffractogramme DRX calculé et mesuré du sel racémique de Bahma de l'exemple 1.

**La Figure 7** représente l'isotherme isobare ternaire du système {Enantiomère (R) de Bahma - Enantiomère (S) de Bahma - Solvant} illustrant le procédé de purification énantiomérique de la présente invention.

**La Figure 8** est une comparaison des diffractogrammes, obtenus par analyse de diffraction des rayons X, de la forme B du (R)(-) Baclofène et du Test 1 de l'exemple 5.

**La Figure 9** correspond au spectre RMN$^1$H du Test 1 de l'exemple 5 dans le DMSO deutéré.

**La Figure 10** est une comparaison des diffractogrammes, obtenus par analyse de diffraction des rayons X, de la forme B du (R)(-) Baclofène et du Test 2 de l'exemple 5.

**La Figure 11** correspond au spectre RMN$^1$H du Test 2 de l'exemple 5 dans le DMSO deutéré.

**La Figure 12** est une comparaison des diffractogrammes, obtenus par analyse de diffraction des rayons X, de la forme B du (R)(-) Baclofène et du Test 3 de l'exemple 5.

**La Figure 13** correspond au spectre RMN$^1$H du Test 3 de l'exemple 5 dans le DMSO deutéré.

**La Figure 14** correspond au spectre RMN$^1$H du sel d'hydrogénomaléate de baclofène dans l'eau deutérée.

**La Figure 15** correspond au spectre RMN$^1$H du baclofène dans l'eau deutérée.

**La Figure 16** correspond au spectre RMN$^1$H de l'acide Maléique dans l'eau deutérée.

## TECHNIQUES ANALYTIQUES

### Détermination du point de fusion/décomposition et obtention du diagramme de phase binaire par calorimétrie différentielle à balayage (DSC)

Les mesures calorimétriques différentielles à balayage ont été réalisées de la façon suivante :

- DSC 204 F1 Netzsch équipé d'un Intracooler
- Creuset Aluminium, couvercle Aluminium fermé
- Atmosphère: Helium
- Vitesse de chauffe: 5K. min$^{-1}$
- Traitement des données: logiciel Netzsch Proteus Thermal Analysis (v.4. 8. 4)

Suite aux différentes analyses DSC et HPLC chirale réalisées sur des monocristaux obtenus à 20 et 70°C (98.3%ee à 70°C et 98.8%ee à 20°C), le diagramme de phase binaire de la Figure 1 a été établi. L'excès énantiomérique (%ee) a été déterminé par HPLC chirale selon la méthode décrite ci-dessous.

### Détermination de l'excès énantiomérique (%ee) par HPLC chirale

La méthode chromatographique provient de celle décrite dans Hefnawy, M., Aboul-Enein, H. Talanta, 2003, vol. 61, n°5, pp. 667-673.

Les excès énantiomériques ont été déterminés par chromatographie HPLC chirale à l'aide d'une colonne Chirobiotic T (longueur 15 cm, diamètre interne 4,6 mm, particules 5 μm) montée sur une chaine HPLC Spectra System munie d'un passeur d'échantillon AS, d'une pompe P1000 et d'un détecteur UV1000. Les conditions expérimentales étaient :

- Solvant : mélange isocratique de méthanol, d'eau, d'acide acétique et de triéthylamine en proportion 98 : 2 : 0.1 : 0.1 ;
- Débit : 1 ml.min$^{-1}$;
- Détecteur : λ = 226 nm ;
- Volume injecté : 10 μL

### Détermination de l'excès énantiomérique (%ee) par polarimétrie

Entre chaque cristallisation préférentielle, les excès énantiomérique (%ee) des précipités et de la solution ont également été déterminés par polarimétrie. Cette technique est plus rapide que l'analyse HPLC chirale et permet donc de vérifier le bon déroulement du procédé de dédoublement afin d'ajuster les paramètres en conséquence (quantité de solvant et de sel racémique de Bahma à compenser avant le début d'une cristallisation).

Ces analyses ont été effectuées sur un polarimètre Perkin Elmer Model 341 muni d'une cellule de mesure de 10 cm thermostatée et permettant l'analyse à différentes longueurs d'onde. Les mesures ont été faites à 25°C et les échantillons ont été dissous dans l'azéotrope eau/n-propanol (43.29 %mol). Le tableau ci-dessous donne le pouvoir rotatoire spécifique (a) d'un énantiomère pur de Bahma à différentes longueurs d'ondes (λ).

| λ (nm) | α (°) |
|---|---|
| **365** | **-0.35** |
| 589 | -0.11 |
| 578 | -0.08 |
| 546 | -0.1 |
| 436 | -0.19 |

La longueur d'onde de 365 nm a été retenue car elle présentait la meilleure déviation de la lumière polarisée (-0.35°).

La Figure 5 montre la droite d'étalonnage qui a été tracée en faisant varier la concentration de l'énantiomère pur de Bahma et en mesurant le pouvoir rotatoire spécifique à une longueur d'onde de 365 nm. Les valeurs sont reportées dans le tableau ci-après.

| C (g/dL) | α (°) |
|---|---|
| 2.20 | -0.34 |
| 1.65 | -0.26 |
| 1.24 | -0.22 |
| 0.93 | -0.16 |
| 0.70 | -0.12 |
| 0.00 | 0 |

On a alors pu déduire la valeur du pouvoir rotatoire spécifique de Bahma avec la formule suivante:

$$\alpha = [\alpha]_{365nm}^{25°C} * l * C$$

dans laquelle :

α est le pouvoir rotatoire de l'échantillon en degrés (°) ;
C est la concentration de l'échantillon en $g.dL^{-1}$;
I est la longueur de la cellule d'analyse en dm ;
$[\alpha]_{365nm}^{25°C}$ est le pouvoir rotatoire spécifique de Bahma à 25°C et à 365 nm dans le solvant utilisé exprimé en $°.dL.g^{-1}.dm^{-1}$.

Le pouvoir rotatoire spécifique de Bahma dans ces conditions est de 0.1642 $°dL.g^{-1}.dm^{-1}$.

## Analyse par diffraction des rayons X sur monocristal

Le monocristal choisi a été collé au bout d'une baguette de verre et monté sur une tête goniométrique du diffractomètre Bruker SMART APEX muni d'un détecteur bidimensionnel. Trois ensembles de mesures ont été enregistrés (au total 1800 clichés (frames)) correspondant à 3 ω balayages (scans) (incrémentation de 0.3°), pour quatre valeurs différentes de ϕ.

Les paramètres de la maille élémentaire et la matrice d'orientation ont été déterminés en utilisant le programme SMART. Les intégrations des données et l'affinement des paramètres de maille ont été réalisés à l'aide du programme SAINT. Les intensités ont été corrigées du facteur de polarisation de Lorentz et d'absorption par les programmes SAINT et SADABS pour obtenir les $F_O^2$.(hkl). Le programme WinGX a été utilisé pour la détermination du groupe d'espace, la résolution de la structure et son affinement.

## Analyse par diffraction des rayons X sur poudres

Les analyses de diffraction des rayons X sur poudres ont été réalisées avec un diffractomètre D8 Discover (Bruker). L'instrument est équipé d'un tube à rayons X contenant une anticathode de cuivre (40kV, 40 mA, radiation $K\alpha 1$ = 1.5406 Å, radiation $K\alpha 2$ = 1.5444 Å) et est monté avec un détecteur angulaire Lynx eye. Le programme d'analyse utilisé est un balayage 3 à 30° en 2θ par pas de 0.04° avec 0.5s/pas et une rotation de 20rpm (Phi spinner).

## Détermination de solubilité

La solubilité d'un sel de Bahma dans un solvant donné a été calculée, pour une température donnée et dans un volume de solvant donné, avec la formule suivante:

$$\frac{m_{Bahma}}{m_{Bahma} + (\rho_{solvant} \times V_{solvant})} \times 100$$

dans laquelle

- $m_{Bahma}$ est la masse du sel de Bahma introduite en grammes pour atteindre la saturation ;
- $\rho_{solvant}$ est la densité du solvant en $g.mL^{-1}$ ; et
- $V_{solvant}$ est le volume du solvant en mL.

## Dispositif expérimental pour le dédoublement par cristallisation préférentielle

Les cristallisations ont été effectuées dans des tubes fermés (diamètre 3cm, longueur 9cm). L'agitation était assurée par des barreaux aimantés en forme de croix et le contrôle en température par un cryothermostat programmable Lauda ECO RE 415.

Les entrainements ont été mis en œuvre par le procédé AS3PC décrit dans la demande de brevet WO 1995/008522.

Au cours des entrainements, des échantillons des solutions (10 µL dilué dans 1mL de phase mobile) ont été prélevés afin de déterminer leurs excès énantiomérique par chromatographie selon la méthode décrite ci-dessus.

### a) Première cristallisation

On a préparé un volume V de 40mL de solution de sel racémique de Bahma saturée à 50°C (température $T_L$) dans un solvant ou un mélange de solvant par filtration d'une suspension à cette même température après un temps d'équilibration de plusieurs heures pour atteindre la saturation.

A cette solution limpide sont ajoutés au moins 5% en poids d'excès d'un énantiomère pur de Bahma (Bahma-100%ee) par rapport au poids du sel de Bahma racémique (Bahma rac.) introduit. La suspension obtenue est ensuite légèrement surchauffée à une température $T_B = T_L$ +3°C. Ainsi, tous les germes de l'énantiomère en défaut pouvant subsister à $T_L$ sont nécessairement dissous. Le système de départ est donc une suspension de l'énantiomère en excès. La phase liquide de la suspension est saturée en un énantiomère et légèrement sous-saturée en l'autre énantiomère. Ce système présente l'avantage d'être à l'équilibre thermodynamique.

Une rampe de température de refroidissement est ensuite appliquée au système de $T_B$ à $T_F$ ($T_F < T_B$), température finale à laquelle le système est rapidement filtré sans attendre l'établissement de l'équilibre thermodynamique.

### b) Cristallisations suivantes

A la fin de chaque entrainement, les suspensions ont été filtrées sur verre fritté. Un échantillon des filtrats (10 µL de filtrat dilué dans 1mL de phase mobile) a été récupéré pour analyse du %ee par HPLC chirale et le restant a été mis de côté pour réaliser l'entrainement suivant. Le solide récupéré a été pesé puis 15 mg ont été dissous dans 1.5 mL d'azéotrope eau/n-propanol pour analyse du %ee par polarimétrie et 10 µL de cette solution ont été dilués dans 1mL de phase mobile pour analyse du %ee par HPLC chirale.

Le filtrat récupéré a été compensé en ajoutant une masse de sel racémique de Bahma sensiblement égale à celle des cristaux récupérés à la cristallisation précédente. Le filtrat a aussi été compensé des pertes en solvant en ajoutant du solvant jusqu'à atteindre 40 mL (volume de solvant initial).

Le système a ensuite été chauffé de nouveau à $T_B$ où une nouvelle suspension a été obtenue. Après une demi-heure de mise à l'équilibre à cette température, le même programme de refroidissement a été appliqué à la fin duquel

une nouvelle filtration donne l'énantiomère opposé au précédent. Le recyclage successif permet de récupérer le même énantiomère que celui de départ suite aux cristallisations impaires, alors que l'autre énantiomère est systématiquement récupéré pour toutes les cristallisations paires.

Par recyclage successif, il est alors possible de dédoubler de façon préparative les deux énantiomères d'un mélange racémique.

## EXEMPLES

### Exemple 1 : Préparation et Caractérisation de sels de Bahma

Les sels de Bahma (racémique ou énantiopur) utilisés dans le procédé de la présente invention ont été préparés par évaporation d'une suspension de baclofène (racémique ou énantiopur) et d'acide maléique (mélange de stœchiométrie 1:1) dans de l'acétone.

Les monocristaux de sel de Bahma pour les analyses de diffraction des rayons X ont été obtenus par dissolution de 50mg de sel racémique de Bahma dans un volume donné de solvant : eau, méthanol ou encore azéotrope eau/n-propanol (pour atteindre une solution homogène, le mélange peut être chauffé). Après dissolution des solides, une température peut être imposée à la solution ou celle-ci est laissée à température ambiante (environ 20°C). Les cristaux de sel fortement enrichis de Bahma se forment par évaporation du solvant ou mélange de solvant après quelques jours pour les évaporations les plus lentes, des monocristaux ont ainsi été obtenus par évaporation de solutions laissées à 20, 50 et 70°C.

Ces monocristaux ont été étudiés par diffraction des rayons X pour en déterminer la structure complète. Les données cristallographiques d'un monocristal obtenu à 20°C sont reportées dans le tableau 1.

Tableau 1

| | |
|---|---|
| Système | Monoclinique |
| Groupe d'espace | $P2_1$ (n°4) |
| $a$/Å | 5.728(1) |
| $b$/Å | 13.774(1) |
| $c$/Å | 9.618(9) |
| $\alpha$/° | 90 |
| $\beta$/° | 106.628(1) |
| $\gamma$/° | 90 |
| Volume /Å$^3$ | 727.2(2) |
| Final $R_1$ ($I > 2\sigma(I)$) | 0.0287 |
| Final $wR(F^2)$ ($I > 2\sigma(I)$) | 0.0812 |
| Final $R_1$ | 0.0294 |
| Final $wR(F^2)$ | 0.0817 |
| Paramètre de Flack | -0.02(5) |
| $R_1 = \Sigma (\lVert F_O\rvert - \lvert F_C\rVert) / \Sigma \lvert F_O\rvert$<br>$wR(F^2) = [\Sigma[w(F_O{}^2 - F_C{}^2)^2] / \Sigma[w(F_O{}^2)^2]]^{1/2}$ | |

Le groupe d'espace, le nombre de molécules dans l'unité asymétrique, l'absence de désordre et la valeur du paramètre de Flack indiquent une discrimination chirale quasi-totale à l'état solide à température ambiante. Ces observations ont été corrélées par un comportement identique jusqu'à au moins 70°C.

Le Tableau 2 ci-dessous montre les coordonnées réduites des atomes sauf hydrogène (x$10^4$) et le facteur d'agitation isotrope $U_{éq}$ (Å$^2$ x $10^3$).

Tableau 2

| Atome | x | y | z | $U_{éq}$ |
|---|---|---|---|---|
| C(1) | -5631(3) | 8817(1) | -938(2) | 33(1) |
| C(2) | -3549(3) | 9419(1) | -1112(2) | 35(1) |
| C(3) | -3199(3) | 10379(1) | -272(2) | 31(1) |

(suite)

| Atome | x | y | z | $U_{éq}$ |
|---|---|---|---|---|
| C(4) | -1434(3) | 10998(1) | -828(2) | 36(1) |
| C(5) | -2316(3) | 10237(1) | 1357(2) | 30(1) |
| C(6) | -80(3) | 9806(1) | 2015(2) | 37(1) |
| C(7) | 739(3) | 9687(1) | 3512(2) | 39(1) |
| C(8) | -725(4) | 9994(1) | 4340(2) | 39(1) |
| C(9) | -2951(3) | 10412(1) | 3718(2) | 41(1) |
| C(10) | -3730(3) | 10535(1) | 2229(2) | 35(1) |
| Cl(1) | 289(1) | 9851(1) | 6215(1) | 58(1) |
| N(1) | -1225(3) | 12006(1) | -269(2) | 38(1) |
| O(1) | -7139(2) | 9107(1) | -380(2) | 46(1) |
| O(2) | -5825(2) | 7935(1) | -1485(1) | 39(1) |
| C(1A) | 2665(3) | 2546(1) | 3835(2) | 35(1) |
| O(1A) | 2173(2) | 2425(1) | 2502(1) | 41(1) |
| O(2A) | 4794(3) | 2762(2) | 4591(2) | 67(1) |
| C(2A) | 683(3) | 2437(1) | 4532(2) | 35(1) |
| C(3A) | 787(3) | 2427(2) | 5927(2) | 37(1) |
| C(4A) | 2891(4) | 2502(2) | 7252(2) | 40(1) |
| O(3A) | 5022(3) | 2668(2) | 7127(2) | 62(1) |
| O(4A) | 2565(3) | 2399(1) | 8443(2) | 60(1) |

Le Tableau 3 ci-dessous montre les coordonnées réduites des atomes d'hydrogène ($x10^4$) et le facteur d'agitation isotrope $U_{éq}$ ($Å^2$ x $10^3$).

Tableau 3

| Atome | x | y | z | $U_{éq}$ |
|---|---|---|---|---|
| H(2A) | -3817 | 9558 | -2133 | 41 |
| H(2B) | -2063 | 9042 | -787 | 41 |
| H(3) | -4770 | 10714 | -506 | 38 |
| H(4A) | 161 | 10697 | -542 | 43 |
| H(4B) | -1985 | 11014 | -1880 | 43 |
| H(6) | 883 | 9593 | 1445 | 44 |
| H(7) | 2245 | 9406 | 3947 | 47 |
| H(9) | -3924 | 10610 | 4290 | 49 |
| H(10) | -5231 | 10823 | 1806 | 42 |
| H(1A) | -2693 | 12278 | -496 | 56 |
| H(1B) | -260 | 12345 | -665 | 56 |
| H(1C) | -598 | 11999 | 690 | 56 |
| H(2) | -4657 | 7823 | -1791 | 58 |
| H(2A1) | -867 | 2364 | 3894 | 42 |

(suite)

| Atome | x | y | z | $U_{éq}$ |
|---|---|---|---|---|
| H(3A) | -713 | 2362 | 6108 | 44 |
| H(3A1) | 4939 | 2737 | 6267 | 93 |

Le tableau 4 ci-dessous montre la position et l'intensité calculées et mesurées des pics caractéristiques DRX du sel racémique de Bahma. Les diffractogrammes DRX correspondants sont représentés à la Figure 6.

Tableau 4

| **Indices de Miller** | | | **Bahma calculé** | | | **Bahma mesuré** | | | |
|---|---|---|---|---|---|---|---|---|---|
| **h** | **k** | **l** | **2θ/deg** | **d/Å** | **I/rel.** | **2θ/deg** | **d/Å** | **Intensité (coups)** | **Intensité (I/Io %)** |
| 0 | 0 | 1 | 9.59 | 9.22 | 2.41 | 9.58 | 9.223 | 344 | 1.8 |
| 0 | 1 | 1 | 11.54 | 7.66 | 2.72 | 11.51 | 7.679 | 1116 | 5.8 |
| 0 | 2 | 0 | 12.83 | 6.89 | 5.77 | 12.81 | 6.904 | 2266 | 11.8 |
| 0 | 2 | 1 | 16.04 | 5.52 | 4.96 | 16.03 | 5.525 | 2047 | 10.6 |
| 1 | 0 | 0 | 16.12 | 5.49 | 5.4 | | | | |
| -1 | 0 | 1 | 16.24 | 5.45 | 54.44 | 16.21 | 5.462 | 8893 | 46.2 |
| 1 | 1 | 0 | 17.36 | 5.1 | 33.95 | 17.35 | 5.108 | 6557 | 34 |
| -1 | 1 | 1 | 17.47 | 5.07 | 19.97 | 17.45 | 5.078 | 3944 | 20.5 |
| 0 | 1 | 2 | 20.3 | 4.37 | 6.49 | 20.28 | 4.375 | 1874 | 9.7 |
| 1 | 2 | 0 | 20.66 | 4.3 | 51.05 | 20.64 | 4.3 | 8915 | 46.3 |
| -1 | 2 | 1 | 20.75 | 4.28 | 44.13 | 20.7 | 4.288 | 8204 | 42.6 |
| 1 | 0 | 1 | 21.04 | 4.22 | 3.14 | 21.02 | 4.224 | 1696 | 8.8 |
| 0 | 3 | 1 | 21.59 | 4.11 | 22.72 | 21.56 | 4.119 | 5218 | 27.1 |
| 1 | 1 | 1 | 22.02 | 4.03 | 29.17 | 21.99 | 4.039 | 6216 | 32.3 |
| -1 | 1 | 2 | 22.28 | 3.99 | 17.94 | 22.24 | 3.993 | 2895 | 15 |
| 0 | 2 | 2 | 23.2 | 3.83 | 2.46 | 23.16 | 3.837 | 1381 | 7.2 |
| 1 | 2 | 1 | 24.72 | 3.6 | 14.87 | 24.69 | 3.603 | 3047 | 15.8 |
| -1 | 2 | 2 | 24.95 | 3.57 | 13.08 | 24.92 | 3.57 | 2218 | 11.5 |
| 1 | 3 | 0 | 25.24 | 3.53 | 78.2 | 25.22 | 3.528 | 9331 | 48.4 |
| -1 | 3 | 1 | 25.32 | 3.51 | 7.46 | | | | |
| 0 | 4 | 0 | 25.82 | 3.45 | 100 | 25.79 | 3.452 | 19184 | 100 |
| 0 | 3 | 2 | 27.38 | 3.25 | 11.56 | 27.35 | 3.258 | 2470 | 12.8 |
| 0 | 4 | 1 | 27.61 | 3.23 | 34.09 | 27.57 | 3.233 | 5707 | 29.6 |
| 1 | 0 | 2 | 28.6 | 3.12 | 2.89 | 28.55 | 3.124 | 1238 | 6.4 |
| 1 | 3 | 1 | 28.7 | 3.11 | 6.3 | 28.68 | 3.11 | 1618 | 8.4 |
| -1 | 3 | 2 | 28.9 | 3.09 | 4.41 | 28.87 | 3.09 | 1396 | 7.2 |
| -1 | 0 | 3 | 28.93 | 3.08 | 2.51 | | | | |
| 1 | 1 | 2 | 29.34 | 3.04 | 27.72 | 29.29 | 3.047 | 5113 | 26.5 |
| -1 | 1 | 3 | 29.66 | 3.01 | 4.39 | | | | |
| 0 | 1 | 3 | 29.77 | 3 | 17.51 | 29.71 | 3.005 | 3125 | 16.2 |

**Exemple 2 : Dédoublement dans le mélange azéotropique n-propanol/eau par cristallisation préférentielle autoensemencée**

La solubilité du sel racémique de Bahma à différentes températures a été déterminée dans le mélange azéotropique n-propanol/eau (ρ =0,870 g.mL$^{-1}$). Les valeurs calculées sont introduites dans le tableau ci-dessous.

| Température | Solubilité |
|---|---|
| 20°C | 1,49% |
| 35°C | 2,80% |
| 50°C | 4,81% |

Plusieurs entrainements ont été réalisés dans ce solvant à partir d'une solution racémique saturée à 50°C en suivant le dispositif expérimental décrit précédemment.

- 1ère série:

| **Système Initial:** | **40mL saturés à 50°C (1.7583g de Bahma rac. dans 34.796g de Solvant) et 0.2505g de Bahma-100%ee** | | | | |
|---|---|---|---|---|---|
| | **Température (°C)** | **Temps (min)** | **Solution** | **Récoltes Brutes** | |
| | | | **ee (%)** | **Masse (g)** | **ee (%)** |
| **Essai 1** | 53 | 0 | -4.71 | | |
| | 50 | 6 | -4.44 | | |
| | 45 | 16 | -0.40 | | |
| | 40 | 26 | 4.54 | | |
| | 35 | 36 | 11.69 | | |
| | 32.5 | 41 | 15.30 | | |
| | 30 | 46 | 19.92 | **0.6682 g** | **-91.08** |
| **4h à 53°C** | | | **Compensation 0.6895g de Bahma rac. et 2mL de Solvant** | | |
| **Essai 2** | 53 | 0 | 6.43 | | |
| | 35 | 36 | -9.89 | | |
| | 30 | 46 | -18.08 | **0.6587 g** | **+84.29** |
| **12h à 53°C** | | | **Compensation 0.6718g de Bahma rac. et 2 mL de Solvant** | | |
| **Essai 3** | 53 | 0 | -5.47 | | |
| | 35 | 36 | -3.37 | | |
| | 30 | 46 | 10.02 | **0.4971 g** | **-91.03** |
| **2h à 53°C** | | | **Compensation 0.4955g de Bahma rac. et 2mL deSolvant** | | |
| **Essai 4** | 53 | 0 | 5.14 | | |
| | 35 | 36 | -2.38 | | |
| | 30 | 46 | -11.27 | **0.4156 g** | **+82.72** |

(suite)

| 1h à 53°C | | | Compensation 0.4153g de Bahma rac. | | |
|---|---|---|---|---|---|
| **Essai 5** | 53 | 0 | -3.07 | | |
| | 35 | 36 | 12.84 | | |
| | 30 | 46 | 20.93 | **0.5608 g** | **-93.78** |
| **30 min à 53°C** | | | **Compensation 0.555g de Bahma rac. et 1.5mL deSolvant** | | |
| **Essai 6** | 53 | 0 | 9.76 | | |
| | 35 | 36 | -5.35 | | |
| | 30 | 46 | -13.76 | **0.5339 g** | **+96.59** |

- 2ème série :

| **Système Initial:** | **40mL saturés at 50°C (1.7583g de Bahma rac. dans 34.796g de Solvant) et 0.2445g de Bahma-100%ee** | | | | |
|---|---|---|---|---|---|
| | **Température (°C)** | **Temps (min)** | **Solution** | **Récoltes brutes** | |
| | | | **ee (%)** | **Masse (g)** | **ee (%)** |
| **Essai 1** | 53 | 0 | -11.88 | | |
| | 35 | 36 | -3.47 | | |
| | 30 | 46 | 9.75 | **0.5012 g** | **-94.04%** |
| **60min à 53°C** | | | **Compensation 0.5120g de Bahma rac. et 1.2mL de Solvant** | | |
| **Essai 2** | 53 | 0 | 5.70 | | |
| | 35 | 36 | -9.90 | | |
| | 30 | 46 | -17.87 | **0.4679 g** | **+84.61%** |
| **30 min à 53°C** | | | **Compensation 0.4621g de Bahma rac. et 1mL de Solvant** | | |
| **Essai 3** | 53 | 0 | -8.86 | | |
| | 35 | 36 | 6.62 | | |
| | 30 | 46 | 15.26 | **0.5496 g** | **-95.07%** |
| **30 min à 53°C** | | | **Compensation 0.5469g de Bahma rac.et 1.5mL de Solvant** | | |
| **Essai 4** | 53 | 0 | 8.16 | | |
| | 35 | 36 | -8.64 | | |
| | 30 | 46 | -17.34 | **0.6465g** | **+92.52%** |
| **30 min à 53°C** | | | **Compensation 0.5122g de Bahma rac. et 1.5mL de Solvant** | | |
| **Essai 5** | 53 | 0 | -8.02 | | |
| | 35 | 36 | 9.69 | | |
| | 30 | 46 | 19.47 | **0.6463g** | **-88.51%** |

(suite)

| 30 min à 53°C | | | Compensation 0.5136g de Bahma rac. et 1.75mL de Solvant | | |
|---|---|---|---|---|---|
| **Essai 6** | 53 | 0 | 11.09 | | |
| | 35 | 36 | -5.15 | | |
| | 30 | 46 | -17.59 | **0.6124g** | **+91.71%** |
| **30 min à 53°C** | | | **Compensation 0.4975g de Bahma rac. et 1.5mL de Solvant** | | |
| **Essai 7** | 53 | 0 | -12.35 | | |
| | 35 | 36 | -2.47 | | |
| | 30 | 46 | 12.65 | **0.5378g** | **-91.71%** |
| **30 min à 53°C** | | | **Compensation 0.5306g de Bahma rac. et 2mL de Solvant** | | |
| **Essai 8** | 53 | 0 | 7.46 | | |
| | 35 | 36 | -4.89 | | |
| | 30 | 46 | -14.73 | **0.5254g** | **+88.90%** |
| **30 min à 53°C** | | | **Compensation 0.5238g de Bahma rac. et 0.5mL de Solvant** | | |
| **Essai 9** | 53 | 0 | -6.25 | | |
| | 35 | 36 | 10.26 | | |
| | 30 | 46 | 19.42 | **0.6444g** | **-89.51%** |
| **30 min à 53°C** | | | **Compensation 0.5183g de Bahma rac. et 1mL de Solvant** | | |
| **Essai 10** | 53 | 0 | 8.00 | | |
| | 35 | 36 | -7.59 | | |
| | 30 | 46 | -16.49 | **0.7194g** | **+87.05%** |

**Exemple 3** : **Dédoublement dans de l'eau acidifiée par cristallisation préférentielle autoensemencée**

La solubilité du sel racémique de Bahma à différentes température a été déterminée dans l'eau pure ($\rho$ =1 g.mL$^{-1}$), dans une solution aqueuse de HCl à 1M ($\rho$ =1,017 g.mL$^{-1}$) et dans une solution aqueuse de HCl à 2M ($\rho$ =1,030 g.mL$^{-1}$). Les valeurs calculées sont introduites dans le tableau ci-dessous.

| Température | Solubilité Eau | Solubilité HCl 1M | Solubilité HCl 2M |
|---|---|---|---|
| 20°C | 0.75% | 4.51% | 6.48% |
| 35°C | 1.00% | 6.86% | 11.44% |
| 50°C | 1.78% | 12.54% | 22.24% |

Ainsi, l'utilisation d'une solution aqueuse acidifiée permet avantageusement d'augmenter la solubilité du sel racémique de Bahma ce qui permet d'améliorer la productivité de la cristallisation préférentielle. Pour des concentrations en HCl de 1M et 2M à ces températures, les phases solides ne contiennent pas de chlorhydrate.

Un entrainement a été réalisé dans HCl 1M à partir d'une solution racémique saturée à 50°C en suivant le dispositif expérimental décrit précédemment.

| **Système Initial:** | **40mL saturés à 50°C (5.8326g de Bahma rac. et 40.68g de HCl 1M) et 0.1735g de Bahma-100%ee** | | | | |
|---|---|---|---|---|---|
| | **Température (°C)** | **Temps** | **Solution** | **Récoltes Brutes** | |
| | | **(min)** | **ee (%)** | **Masse (g)** | **ee (%)** |
| **Essai 1** | 50.25 | 0 | -2.89 | | |
| | 47.5 | 6 | -2.81 | | |
| | 45 | 11 | -2.03 | | |
| | 42.5 | 16 | -0.90 | | |
| | 40 | 21 | 3.76 | | |
| | 37.5 | 26 | 11.55 | **0.8885** | **-90.56%** |

Un entrainement a été réalisé dans HCl 2M à partir d'une solution racémique saturée à 50°C en suivant le dispositif expérimental décrit précédemment.

| **Système initial:** | **40mL saturés à 50°C (11.7835g de Bahma rac. dans 41.2g d'HCl 2M) et 0.2501g de Bahma-100%ee** | | | | |
|---|---|---|---|---|---|
| | **Température (°C)** | **Temps (min)** | **Solution** | **Récoltes brutes** | |
| | | | **ee (%)** | **Masse (g)** | **ee(%)** |
| **Essai 1** | 50.5 | 0 | -3.53 | | |
| | 47.5 | 6 | -2.66 | | |
| | 45.0 | 11 | -2.09 | | |
| | 42.5 | 16 | -5.98 | | |
| | 40.0 | 21 | 3.74 | | |
| | 37.5 | 26 | 10.52 | **1.7437g** | **-89.94%** |
| **60 min à 50.5°C** | | | **Compensation : 1.7457g de Bahma rac. et 2mL HCl 2M** | | |
| **Essai 2** | 50.5 | 0 | / | | |
| | 40.0 | 21 | -2.77 | | |
| | 37.5 | 26 | -11.80 | **2.1713g** | **+98.37%** |

## Exemple 4 : Procédé de purification énantiomérique selon l'invention

Le procédé de purification énantiomérique selon l'invention a été mis en œuvre à partir de 0,4239 g de sel de Bahma à -50.43 %ee, c'est-à-dire un mélange de 0,2138 g de (R)-Bahma et 0,2101 g de mélange racémique Bahma, auquel une masse de 27,0787 g d'eau ($m_{H0}$ = 26,7258 g d'eau, soit un excès $\Delta m$ = 1.32% d'eau) a été ajoutée.

Puis le système a été laissé sous agitation magnétique à 20°C pendant une nuit et la suspension filtrée.

Le solide a ensuite été lavé deux fois à l'eau et la récolte a ainsi pu être analysée. On obtient 0,1905g de sel de (R)-Bahma solide à -98.59 %ee et le filtrat présente une pureté mesurée de -11.56 %ee.

## Exemple 5 : Procédé d'obtention du baclofène pur à partir du sel d'hydrogénomaléate de Baclofène

1g de sel énantiopure d'hydrogénomaléate de Baclofène (correspondant à une masse de 0.6480g de baclofène et 0.3520g d'acide maléique) est dissout dans 10ml d'une solution de NaOH 1M à 25°C et sous agitation.

Le pH de la solution est alors ajusté par ajout d'un volume connu d'une solution d'acide chlorhydrique à 37% massique. La température est parallèlement contrôlée. L'ajout d'acide chlorhydrique entraîne la précipitation de la forme B du baclofène qui est ensuite filtré, séché, pesé et analysé par diffraction des rayons X et par analyse RMN (Résonnance Magnétique Nucléaire).

Trois tests ont été ensuite effectués afin de vérifier que le procédé est viable à différentes valeurs de pH final

et différentes températures.

Test 1 :

| Test | Volume HCl ajouté | Température finale | pH final | Masse récoltée |
|---|---|---|---|---|
| Test 1 | 230 µL | 25°C | 9.02 | 0.5485g |

Test 2 :

| Test | Volume HCl ajouté | Température finale | pH final | Masse récoltée |
|---|---|---|---|---|
| Test 2 | 320 µL | 25°C | 7.90 | 0.5546g |

Test 3:

| Test | Volume HCl ajouté | Température finale | pH final | Masse récoltée |
|---|---|---|---|---|
| Test 3 | 240 µL | 10°C | 9.29 | 0.5786g |

Pour chacun des tests, on réalise des analyses de diffraction des rayons X des solides obtenus ainsi que des analyses RMN (voir figures 8 à 16).

Conclusion :

Les analyses de diffraction des rayons X des solides obtenus démontrent que tous les solides obtenus sont constitués de baclofène énantiopure sous sa forme polymorphique B (voir figures 8, 10 et 12).

Les analyses RMN confirment que les échantillons récupérés sont principalement constitués de baclofène avec quelques possibles traces d'acide maléique restant (pic à 6 ppm) (voir figures 9, 11, 13, 14, 15 et 16).

Les masses récoltées et la pureté indiquent un bon rendement du procédé (comparé à la masse initiale de baclofène dissout). Les tests 2 et 3 indiquent que ce rendement peut être optimisé sans que la pureté soit affectée.

**Revendications**

1. Sel racémique d'hydrogénomaléate de Baclofène (Bahma) **caractérisé en ce qu'**il présente un point de fusion/décomposition de 164±1°C.

2. Utilisation d'un sel d'hydrogénomaléate de Baclofène tel que défini à la revendication 1 pour dédoubler les énantiomères (S) et (R) du Baclofène.

3. Procédé de dédoublement des énantiomères (S) et (R) du Baclofène, **caractérisé en ce que** l'on transforme le Baclofène racémique en sel racémique d'hydrogénomaléate de Baclofène en présence d'acide maléique, puis **en ce que** l'on dédouble ledit sel par cristallisation préférentielle pour séparer les deux énantiomères (S) et (R).

4. Procédé selon la revendication 3, **caractérisé en ce que** le dédoublement du sel racémique est réalisé par cristallisation préférentielle autoensemencée ou par cristallisation préférentielle ensemencée, de préférence par cristallisation préférentielle autoensemencée.

5. Procédé selon la revendication 3 ou 4, **caractérisé en ce que** la cristallisation préférentielle est mise en œuvre avec un solvant choisi parmi un solvant alcoolique, une solution aqueuse, une solution aqueuse acide et les mélanges de ceux-ci.

6. Procédé selon la revendication 3 ou 4, **caractérisé en ce que** la cristallisation préférentielle est mise en œuvre avec une solution aqueuse acide, l'acide étant choisi parmi l'acide chlorhydrique, l'acide acétique, l'acide nitrique, de préférence une solution aqueuse d'acide chlorhydrique, plus préférentiellement une solution aqueuse d'acide chlorhydrique à 2 mol/L.

**7.** Procédé selon l'une quelconque des revendications 3 à 6, **caractérisé en ce que** la cristallisation préférentielle est autoensemencée et **en ce qu'**il comprend les étapes suivantes :

a) on prépare un volume V d'une solution saturée de sel racémique de Bahma dans le solvant à une température $T_L$ ;
b) on ajoute au moins 5% en poids du premier énantiomère Bahma à récupérer par rapport au poids du sel racémique de Bahma ;
c) on chauffe le mélange à une température $T_B = T_L + 3°C$ ;
d) on applique une loi de programmation de refroidissement au mélange de $T_B$ à $T_F$, $T_F$ étant inférieur à $T_B$, telle que le mélange garde une faible sursaturation qui privilégie la croissance du premier énantiomère Bahma présent sous forme de cristaux, tout en interdisant la nucléation spontanée du second énantiomère Bahma dissous dans la solution ;
e) on récolte les cristaux du premier énantiomère Bahma à la température $T_F$ ;
f) on ajoute au mélange sensiblement la même masse de sel racémique de Bahma que la masse de la récolte réalisée à l'étape précédente, on complète avec du solvant pour atteindre le volume V et on porte le nouvel ensemble à la température $T_B$ ;
g) on maintient la température $T_B$ pendant un temps t afin de permettre au système de revenir à l'équilibre thermodynamique ;
h) on applique la même loi de programmation de refroidissement qu'à l'étape (d) au mélange préparé à l'étape (g) contenant le second énantiomère Bahma, de sorte que le mélange garde une faible sursaturation pendant la cristallisation afin de privilégier la croissance du second énantiomère Bahma présent sous forme de cristaux tout en interdisant la nucléation spontanée du premier énantiomère Bahma présent dans la solution ;
i) on récolte les cristaux du second énantiomère Bahma à la température $T_F$ ;
j) on ajoute au mélange sensiblement la même masse de sel racémique de Bahma que la masse de la récolte réalisée à l'étape précédente, on complète avec du solvant pour atteindre le volume V et on porte le nouvel ensemble à la température $T_B$ ;
k) on maintient la température $T_B$ pendant un temps t afin de permettre au système de revenir à l'équilibre thermodynamique ;
l) on répète les étapes (d) à (k) pour obtenir successivement l'un puis l'autre des deux énantiomères.

**8.** Procédé selon la revendication 7, **caractérisé en ce que** la température $T_L$ va de 30 à 70°C, de préférence $T_L$ va de 40 à 60°C, plus préférentiellement $T_L$ est 50°C.

**9.** Procédé de purification énantiomérique de sels de Bahma comprenant la recristallisation de sels de Bahma dans un solvant.

**Patentansprüche**

**1.** Racemisches Salz von Baclofen-Hydrogenmaleat (Bahma), **dadurch gekennzeichnet, dass** es einen Schmelz-/Zersetzungspunkt von $164 \pm 1$ °C aufweist.

**2.** Verwendung eines Salzes von Baclofen-Hydrogenmaleat nach Anspruch 1 zur Spaltung der (S)- und (R)-Enantiomere von Baclofen.

**3.** Verfahren zur Spaltung der (S)- und (R)-Enantiomere von Baclofen, **dadurch gekennzeichnet, dass** das racemische Baclofen in Gegenwart von Maleinsäure in ein racemisches Salz des Baclofen-Hydrogenmaleats umgewandelt wird, dann das genannte Salz durch bevorzugte Kristallisation gespalten wird, um die beiden (S)- und (R)-Enantiomere zu trennen.

**4.** Verfahren nach Anspruch 3, **dadurch gekennzeichnet, dass** die Spaltung des racemischen Salzes durch eine bevorzugte selbstaussäende Kristallisation oder durch eine bevorzugte aussäende Kristallisation, bevorzugt durch eine bevorzugte selbstaussäende Kristallisation erfolgt.

**5.** Verfahren nach Anspruch 3 oder 4, **dadurch gekennzeichnet, dass** die bevorzugte Kristallisation mit einem Lösungsmittel durchgeführt wird, gewählt aus einem alkoholischen Lösungsmittel, einer wässrigen Lösung, einer sauren wässrigen Lösung und deren Mischungen.

6. Verfahren nach Anspruch 3 oder 4, **dadurch gekennzeichnet, dass** die bevorzugte Kristallisation mit einer sauren wässrigen Lösung durchgeführt wird, wobei die Säure gewählt ist aus Salzsäure, Essigsäure, Salpetersäure, bevorzugt einer wässrigen Lösung von Salzsäure, noch stärker bevorzugt einer wässrigen Lösung von 2 mol/l Salzsäure.

7. Verfahren nach einem der Ansprüche 3 bis 6, **dadurch gekennzeichnet, dass** die bevorzugte Kristallisation selbstaussäend ist und dass sie die folgenden Schritte umfasst:

a) Herstellen eines Volumens V einer gesättigten Lösung eines racemischen Bahma-Salzes in dem Lösungsmittel bei einer Temperatur $T_L$;
b) Zugeben von wenigstens 5 Gew.-% des ersten zu gewinnenden Bahma-Enantiomers, bezogen auf das Gewicht des racemischen Bahma-Salzes;
c) Erhitzen der Mischung auf eine Temperatur $T_B = T_L + 3°C$;
d) Anwenden eines Kühlprogrammierungsgesetzes auf die Mischung von $T_B$ und $T_F$, wobei $T_F$ kleiner $T_B$ ist, so dass die Mischung eine leichte Übersättigung behält, die das Wachstum des ersten in Kristallform vorliegenden Bahma-Enantiomers begünstigt, während die spontane Nukleation des zweiten in der Lösung gelösten Bahma-Enantiomers verhindert wird;
e) Ernten der Kristalle des ersten Bahma-Enantiomers bei der Temperatur $T_F$;
f) Hinzufügen der gleichen Masse an racemischem Bahma-Salz zu der Mischung, wie die Masse der im vorherigen Schritt vorgenommenen Ernte; es wird mit Lösungsmittel ergänzt, um das Volumen V zu erreichen, und die neue Zusammensetzung wird auf die Temperatur $T_B$ gebracht;
g) Halten der Temperatur $T_B$ über eine Zeit t, um dem System die Rückkehr zum thermodynamischen Gleichgewicht zu ermöglichen;
h) Anwenden desselben Kühlprogrammierungsgesetzes wie in Schritt (d) auf die in Schritt (g) hergestellte Mischung, die das zweite Bahma-Enantiomer enthält, so dass die Mischung während der Kristallisation eine geringe Übersättigung beibehält, um das Wachstum des in Form von Kristallen vorliegenden zweiten Bahma-Enantiomers zu begünstigen und gleichzeitig die spontane Nukleation des in der Lösung vorliegenden ersten Bahma-Enantiomers zu verhindern;
i) Ernten der Kristalle des zweiten Bahma-Enantiomers bei der Temperatur $T_F$;
j) Hinzufügen im Wesentlichen der gleichen Masse an racemischem Bahma-Salz zu der Mischung, wie die Masse der im vorhergehenden Schritt vorgenommenen Ernte; es wird mit Lösungsmittel ergänzt, um das Volumen V zu erreichen, und die neue Zusammensetzung wird auf die Temperatur $T_B$ gebracht;
k) Halten der Temperatur $T_B$ über eine Zeit t, um dem System die Rückkehr zum thermodynamischen Gleichgewicht zu ermöglichen;
l) Wiederholen der Schritte (d) bis (k), um nacheinander das eine und dann das andere der beiden Enantiomere zu erhalten.

8. Verfahren nach Anspruch 7, **dadurch gekennzeichnet, dass** die Temperatur $T_L$ im Bereich von 30 bis 70°C liegt, bevorzugt $T_L$ im Bereich von 40 bis 60°C liegt und noch stärker bevorzugt $T_L$ gleich 50°C ist.

9. Verfahren zur enantiomeren Reinigung von Bahma-Salzen, umfassend die Umkristallisation von Bahma-Salzen in einem Lösungsmittel.

## Claims

1. A racemic salt of baclofen hydrogen maleate (Bahma), **characterized in that** it has a melting/decomposition point of 164±1 °C.

2. The use of a baclofen hydrogen maleate salt as defined in claim 1 for resolving the (S) and (R) enantiomers of baclofen.

3. A process for resolving the (S) and (R) enantiomers of baclofen, **characterized in that** racemic baclofen is transformed into racemic baclofen hydrogen maleate salt in the presence of maleic acid, and **in that** said salt is then resolved by preferential crystallization to separate the two (S) and (R) enantiomers.

4. The process as claimed in claim 3, **characterized in that** the resolution of the racemic salt is performed by auto-seeded preferential crystallization or by seeded preferential crystallization, preferably by auto-seeded preferential crystallization.

**5.** The process as claimed in claim 3 or 4, **characterized in that** the preferential crystallization is performed with a solvent chosen from an alcoholic solvent, an aqueous solution, an acidic aqueous solution and mixtures thereof.

**6.** The process as claimed in claim 3 or 4, **characterized in that** the preferential crystallization is performed with an acidic aqueous solution, the acid being chosen from hydrochloric acid, acetic acid and nitric acid, preferably an aqueous hydrochloric acid solution, more preferentially an aqueous 2 mol/L hydrochloric acid solution.

**7.** The process as claimed in any one of claims 3 to 6, **characterized in that** the preferential crystallization is auto-seeded and **in that** it comprises the following steps:

a) a volume V of a saturated solution of racemic Bahma salt in a solvent is prepared at a temperature $T_L$;
b) at least 5% by weight of the first Bahma enantiomer to be recovered relative to the weight of the racemic Bahma salt is added;
c) the mixture is heated to a temperature $T_B = T_L + 3°C$;
d) a cooling programming law is applied to the mixture from $T_B$ to $T_F$, $T_F$ being below $T_B$, such that the mixture maintains a low supersaturation which favors the growth of the first Bahma enantiomer present in the form of crystals, while prohibiting the spontaneous nucleation of the second Bahma enantiomer dissolved in the solution;
e) the crystals of the first Bahma enantiomer are harvested at the temperature TF;
f) substantially the same mass of racemic Bahma salt as the mass of the harvest made in the preceding step is added to the mixture, the difference is made up with solvent to reach the volume V and the new combined mixture is brought to the temperature $T_B$;
g) the temperature $T_B$ is maintained for a time t so as to allow the system to return to thermodynamic equilibrium;
h) the same cooling programming law as in step (d) is applied to the mixture prepared in step (g) containing the second Bahma enantiomer, so that the mixture maintains a low supersaturation during the crystallization so as to promote the growth of the second Bahma enantiomer present in the form of crystals while at the same time prohibiting the spontaneous nucleation of the first Bahma enantiomer present in the solution;
i) the crystals of the second Bahma enantiomer are harvested at the temperature $T_F$;
j) substantially the same mass of racemic Bahma salt as the mass of the harvest made in the preceding step is added to the mixture, the difference is made up with solvent to reach the volume V and the new combined mixture is brought to the temperature $T_B$;
k) the temperature $T_B$ is maintained for a time t so as to allow the system to return to thermodynamic equilibrium;
l) steps (d) to (k) are repeated to successively obtain one and then the other of the two enantiomers.

**8.** The process as claimed in claim 7, **characterized in that** the temperature $T_L$ ranges from 30 to 70°C; preferably, $T_L$ ranges from 40 to 60°C and more preferentially $T_L$ is 50°C.

**9.** A process for the enantiomeric purification of Bahma salts, comprising the recrystallization of the Bahma salts in a solvent.

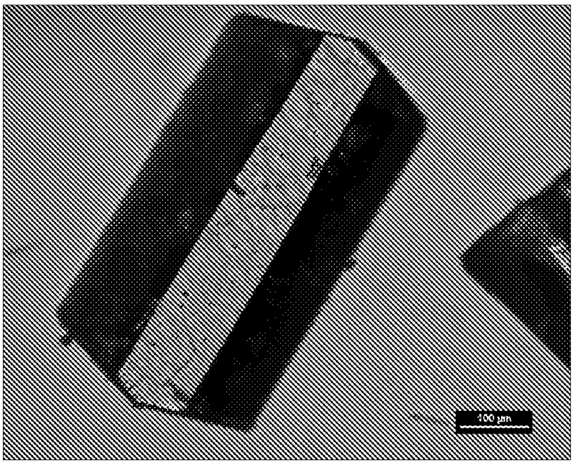


Figure 1

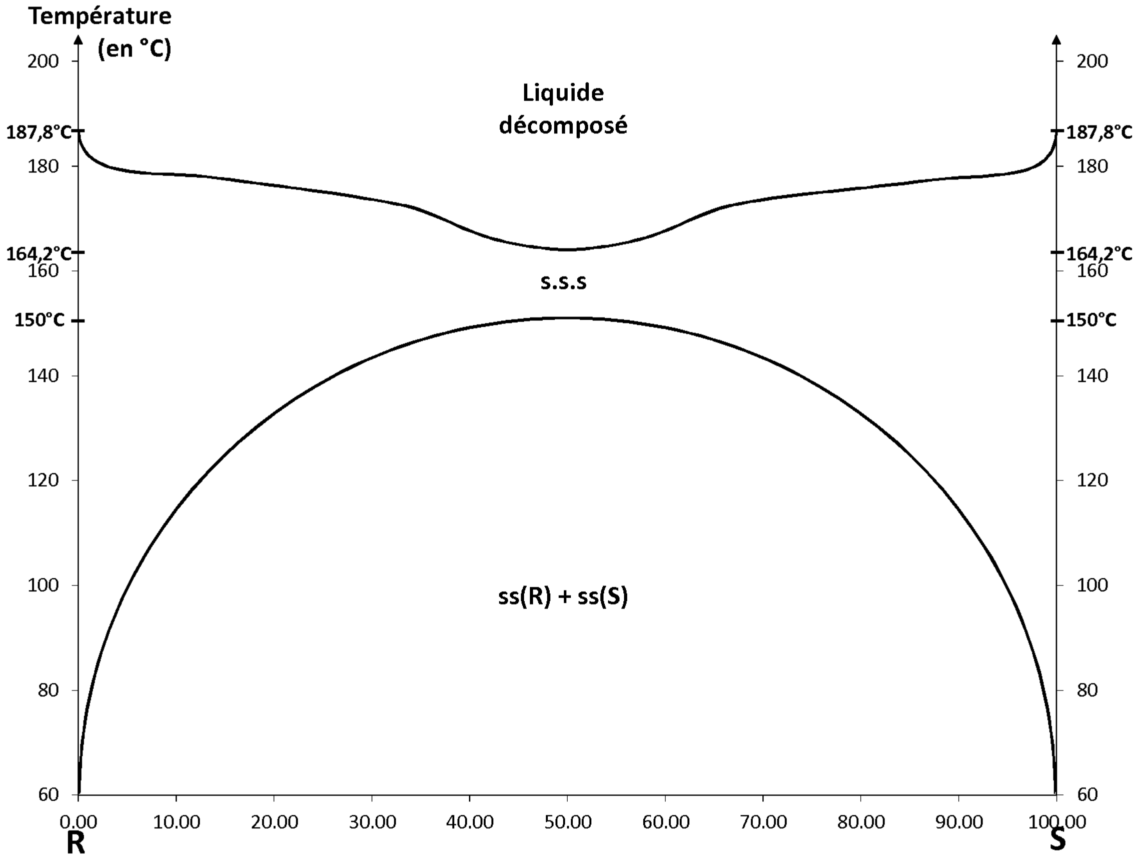


sss : solution solide totale de substitution

ss(S) : solution solide partielle enrichie en énantiomère (S) de Bahma

ss(R) : solution solide partielle enrichie en énantiomère (R) de Bahma

Figure 2

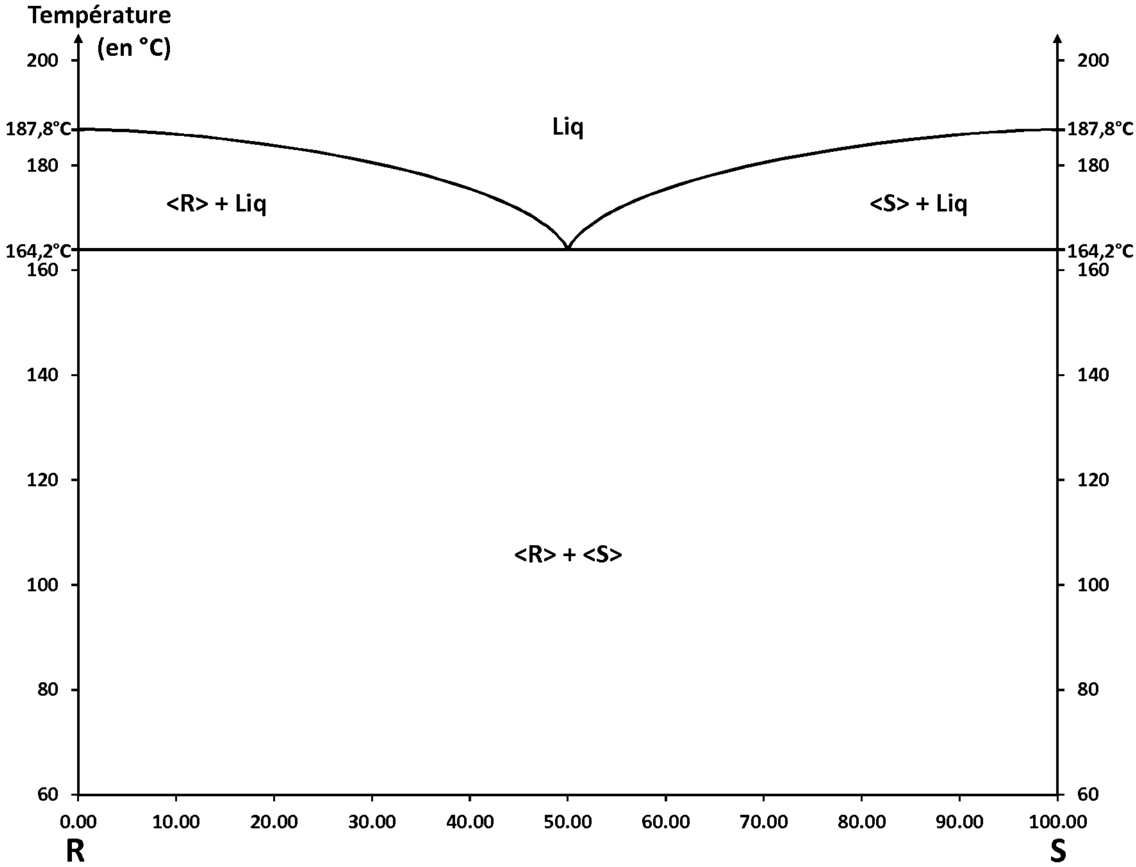


Liq : liquide

<S> : énantiomère (S) sous forme solide

<R> : énantiomère (R) sous forme solide

Figure 3

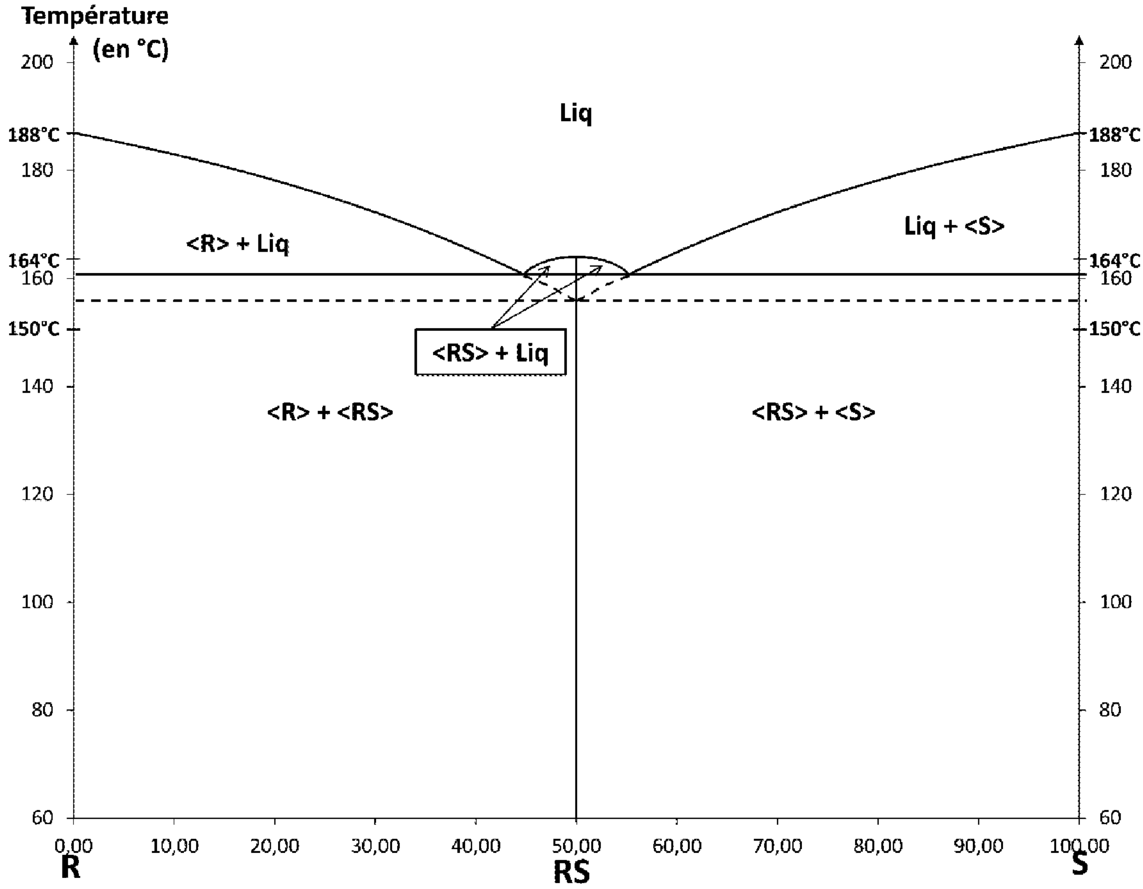


Liq : liquide

<S> : énantiomère (S) sous forme solide

<R> : énantiomère (R) sous forme solide

<RS> : composé racémique sous forme solide

Figure 4

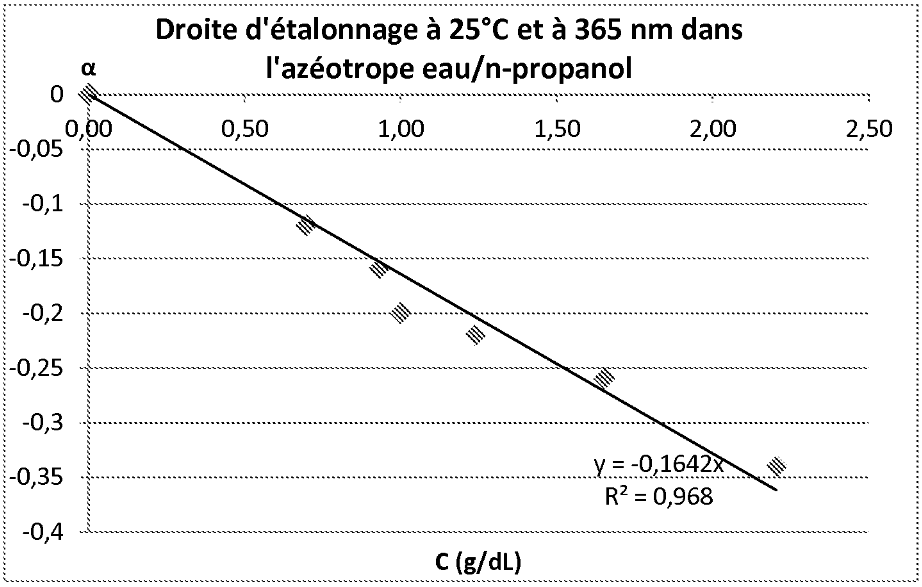


Figure 5

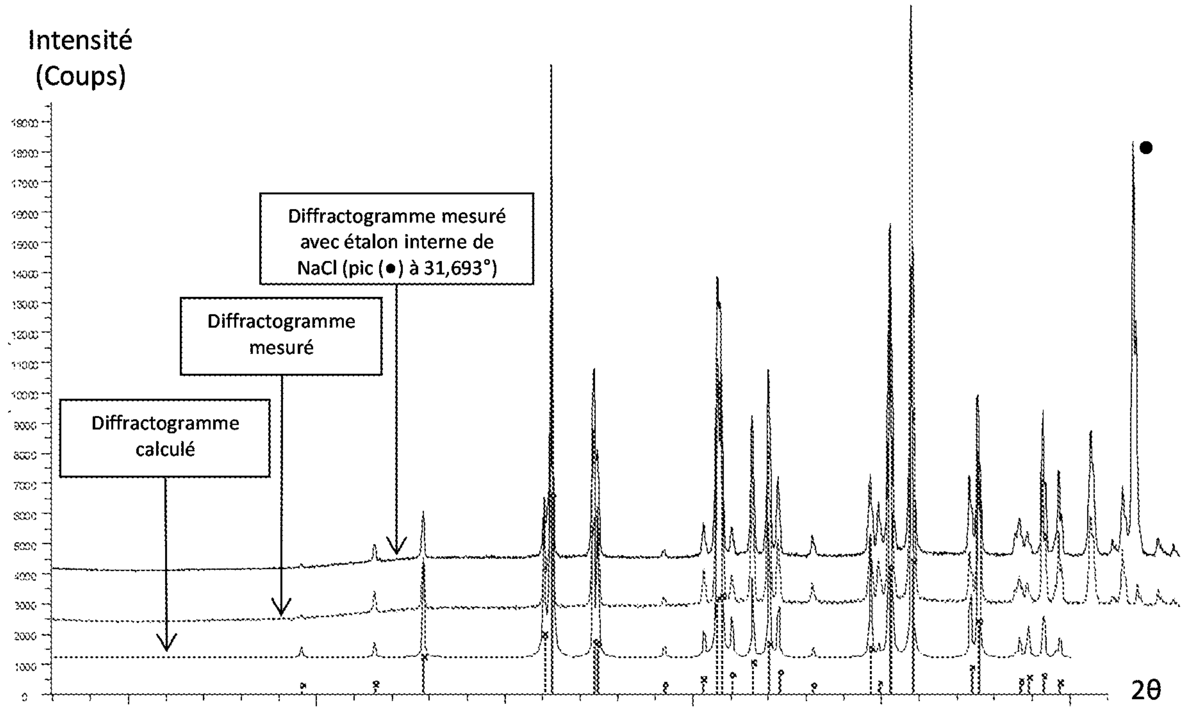


Figure 6

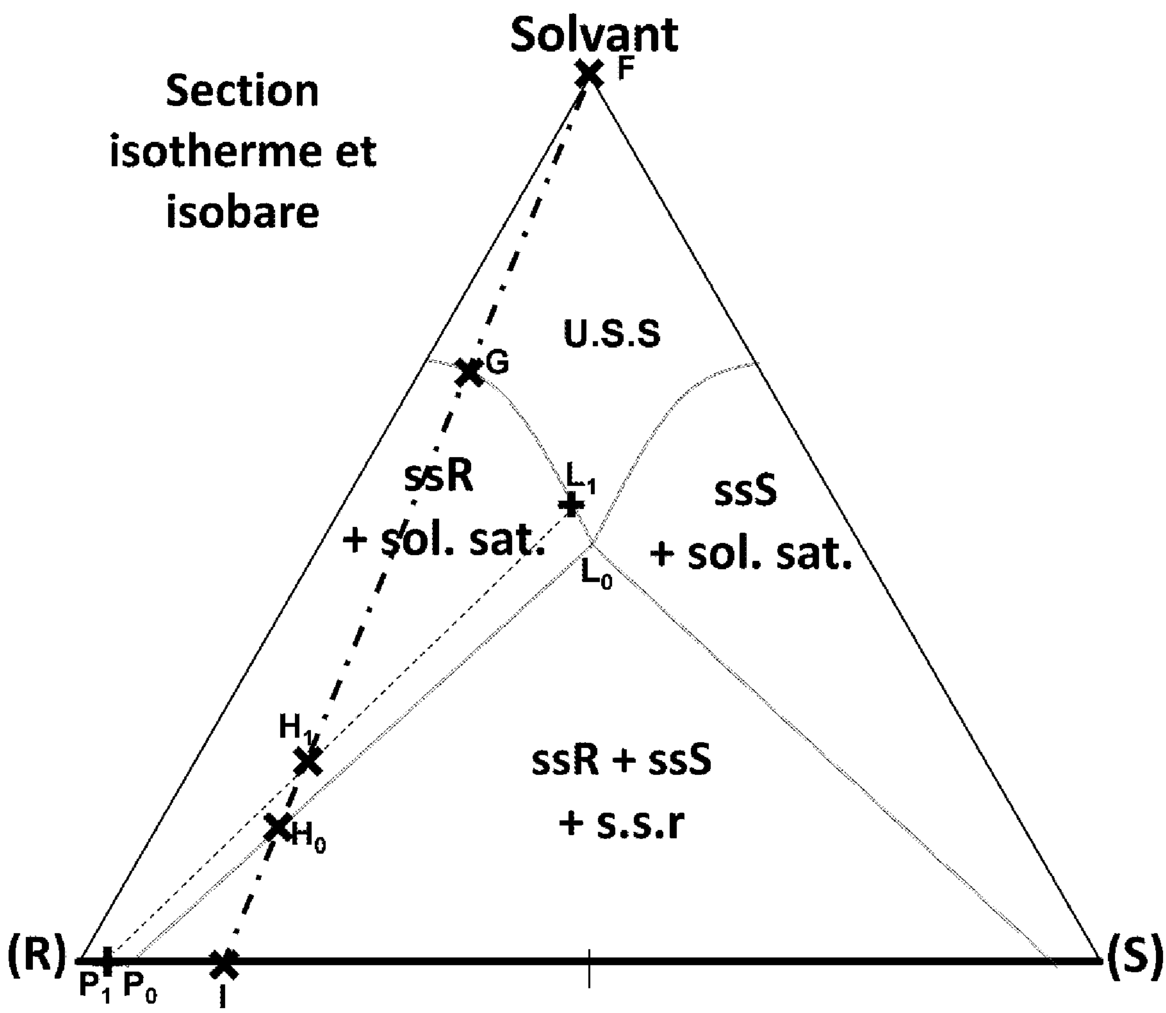


s.s.r. : solution saturée en sel racémique de Bahma

sol. sat. : solution saturée enrichie en énantiomère (S) ou (R) de Bahma

U.S.S : solution sous-saturée

ssR : solution solide partielle enrichie en énantiomère (R) de Bahma

ssS : solution solide partielle enrichie en énantiomère (S) de Bahma

— · — · — · :coupe isoplèthe

$L_0$ = solution racémique saturée

$P_0L_0$ et $P_1L_1$ : conodes

Figure 7

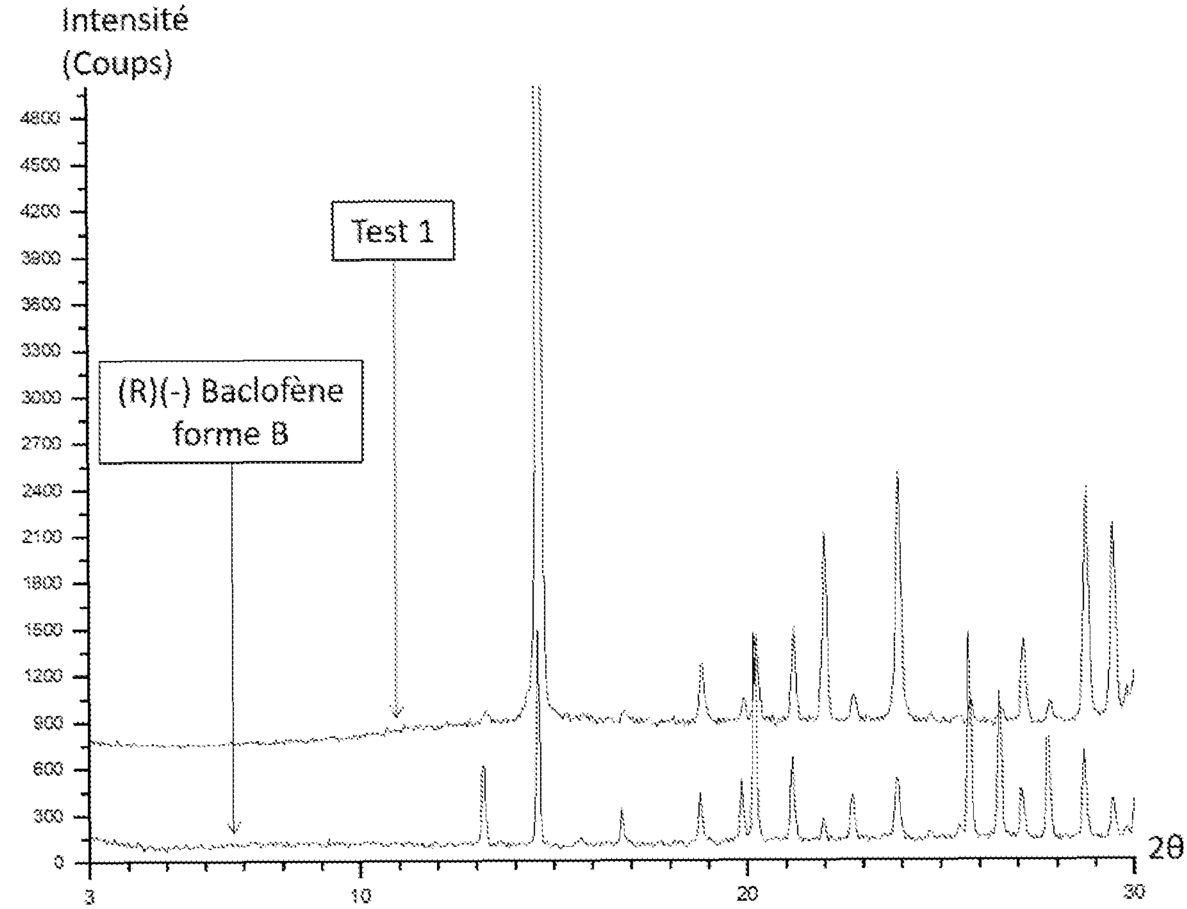


Figure 8

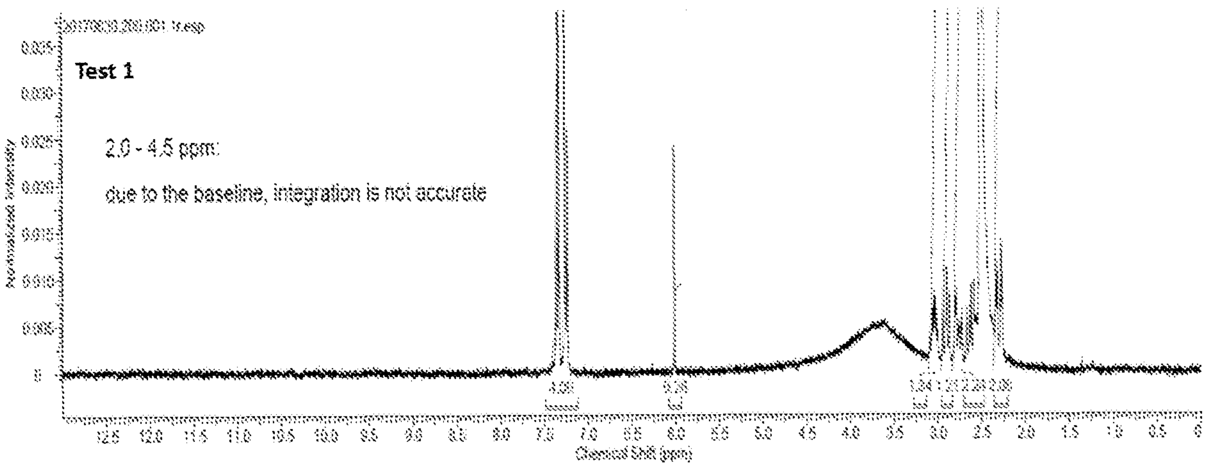


Figure 9

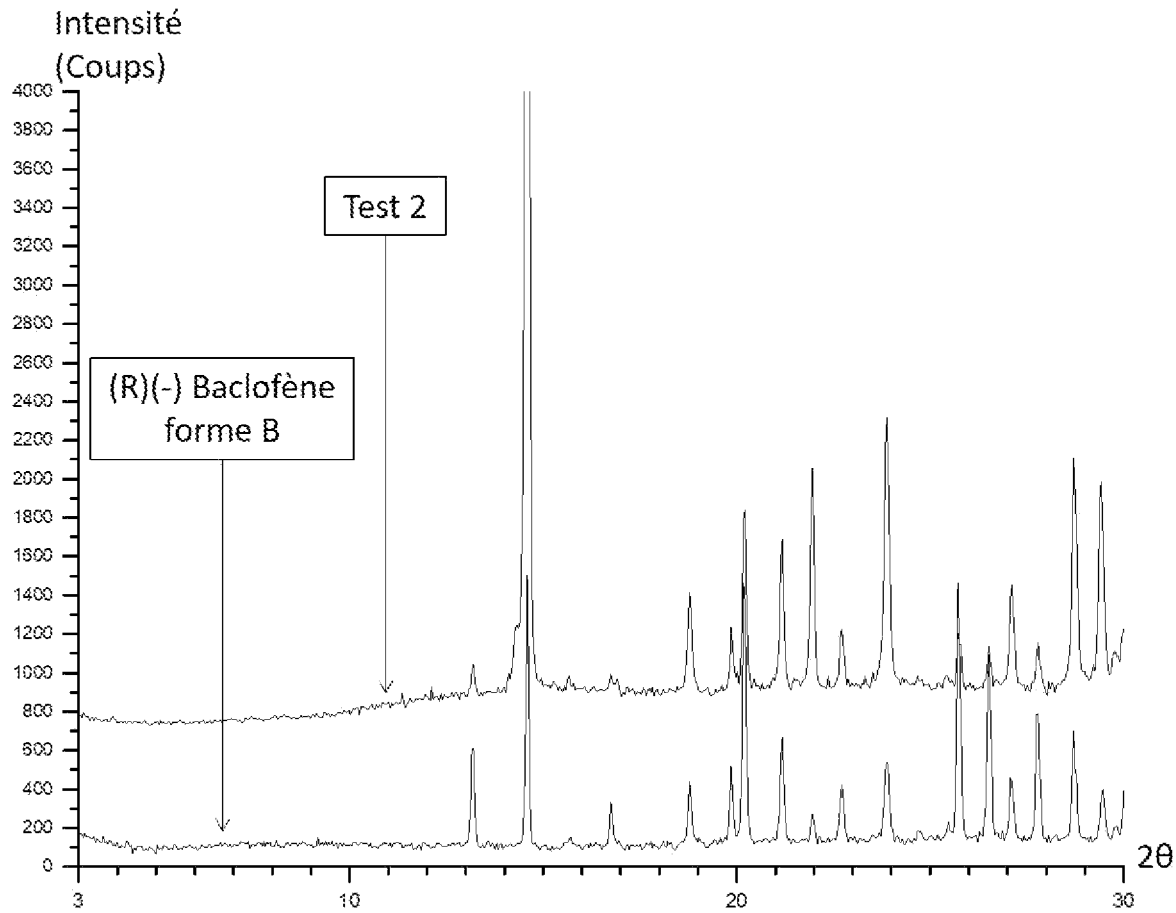


Figure 10

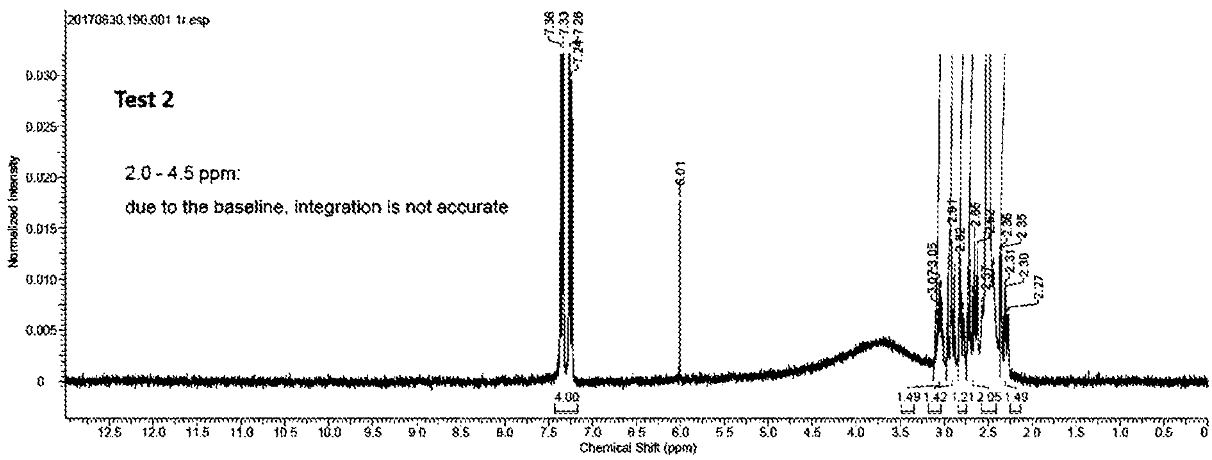


Figure 11

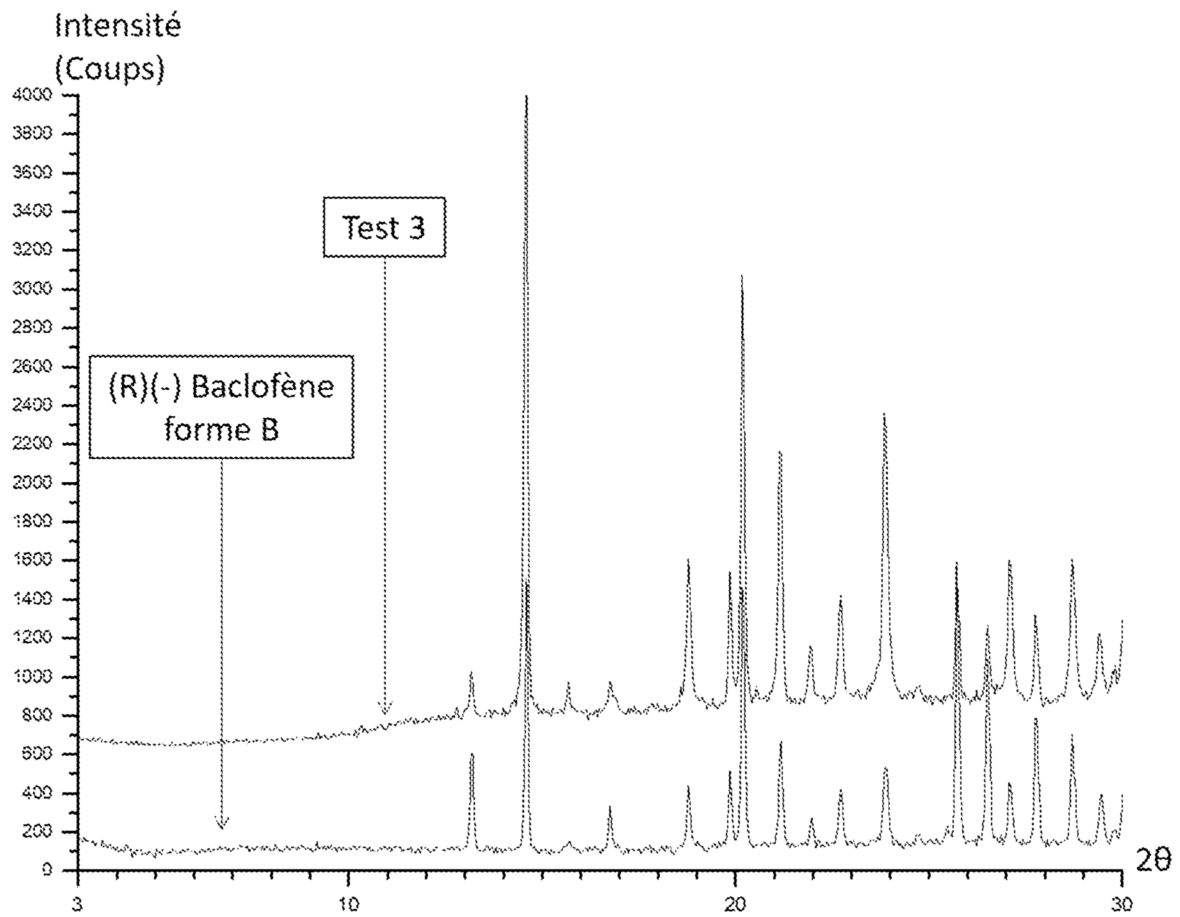


Figure 12

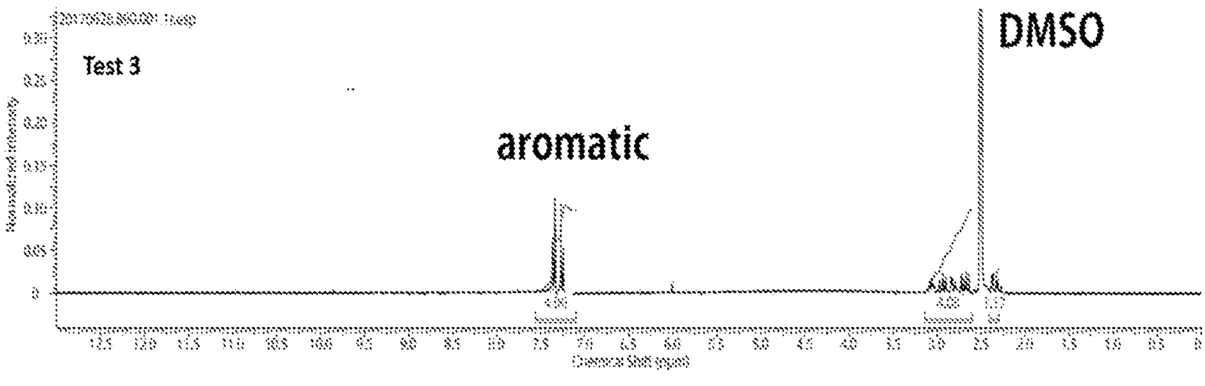


Figure 13

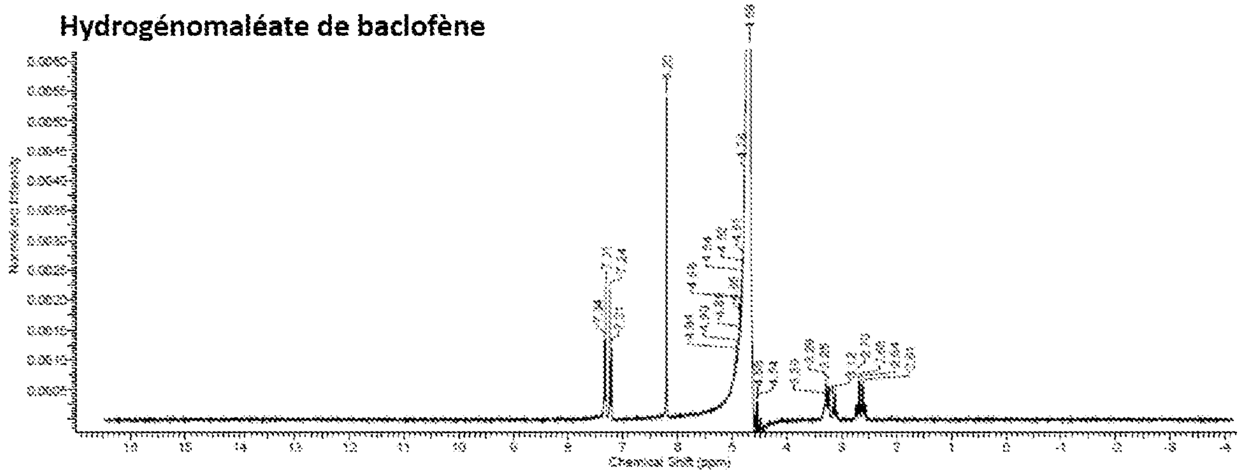


Figure 14

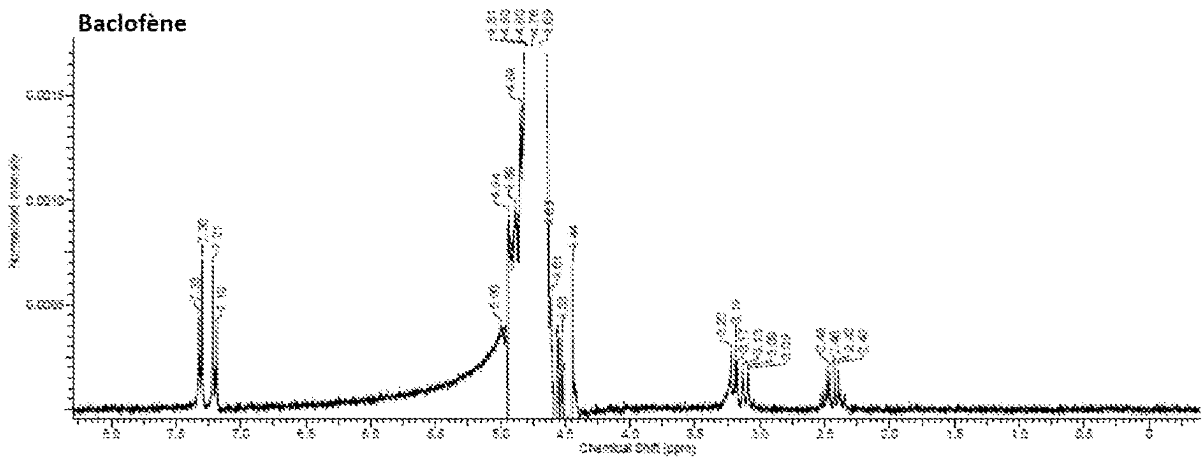


Figure 15

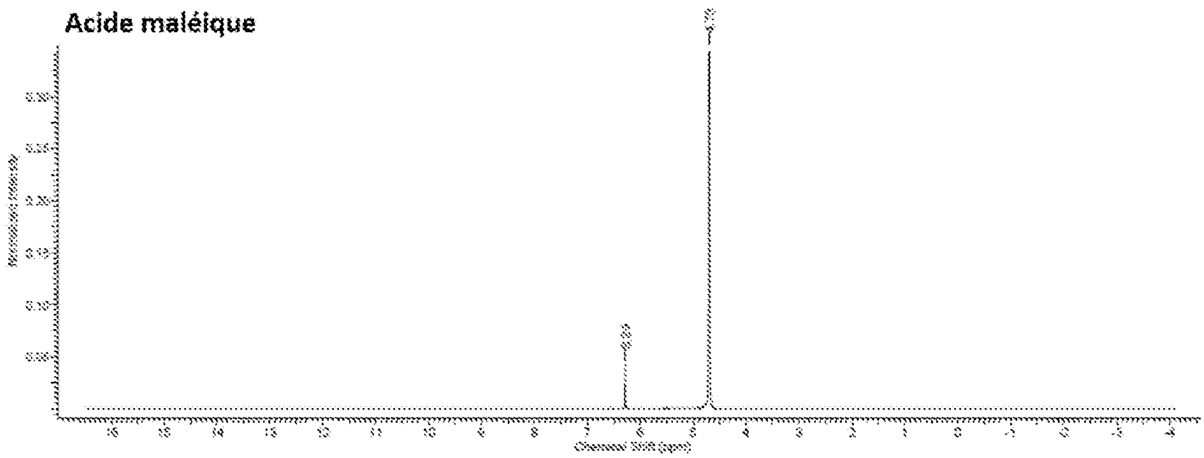


Figure 16

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9402443 A
- FR 2710337
- WO 9508522 A
- EP 0720595 A
- US 6022409 A
- WO 201107330 A
- WO 1995008522 A


### Littérature non-brevet citée dans la description


- JOURNAL OF CONTROLLED RELEASE. ELSEVIER, 01 Juin 1991, vol. 16, 77-88
- **M.X. WANG ; S.M. ZHAO.** *Tetrahedron Lett.,* 2002, vol. 43, 6617-6620
- *Canadian Journal of Chemistry,* 1994, vol. 72 (11), 2312-2317
- **G. COQUEREL.** Preferential Crystallization in Topic in Current Chemistry, Novel Optical Resolution Technologies. Springer, 2007, vol. 269, 1-51
- **HEFNAWY, M. ; ABOUL-ENEIN, H.** *Talanta,* 2003, vol. 61 (5), 667-673